(19) Europäisches Patentamt
European Patent Office
Office européen des brevets

(11) **EP 2 592 999 B1**

(12) **EUROPEAN PATENT SPECIFICATION**

(45) Date of publication and mention of the grant of the patent:
**26.02.2020 Bulletin 2020/09**

(21) Application number: **11807589.4**

(22) Date of filing: **15.07.2011**

(51) Int Cl.:
*A61B 5/02* (2006.01)     *A61B 5/053* (2006.01)

(86) International application number:
**PCT/US2011/044240**

(87) International publication number:
**WO 2012/009669 (19.01.2012 Gazette 2012/03)**

(54) **METHODS AND APPARATUS FOR ESTIMATING A BLOOD VOLUME OF A MAMMALIAN SUBJECT**

VERFAHREN UND VORRICHTUNG ZUR MESSUNG DES BLUTVOLUMENS EINES SÄUGERS

PROCÉDÉS ET APPAREIL POUR ESTIMER UN VOLUME SANGUIN CHEZ UN SUJET MAMMIFÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.06.2011   US 201161495843 P**
            **06.06.2011   US 201161493847 P**
            **16.07.2010   US 365270 P**

(43) Date of publication of application:
**22.05.2013   Bulletin 2013/21**

(73) Proprietor: **Sandgaard, Thomas**
**Castle Rock, Colorado 80104 (US)**

(72) Inventor: **Sandgaard, Thomas**
**Castle Rock, Colorado 80104 (US)**

(74) Representative: **AWA Denmark A/S**
**Strandgade 56**
**1401 Copenhagen K (DK)**

(56) References cited:
**WO-A1-2007/070978      WO-A1-2010/053743**
**US-A- 5 991 654         US-A1- 2005 177 062**
**US-A1- 2005 203 428     US-A1- 2007 032 732**
**US-B2- 7 615 011        US-B2- 7 666 146**

## Description

### CROSS-REFERENCE TO RELATED APPLICATIONS

The present non-provisional application claims priority under 35 U.S.C. § 119(e) to U.S. Provisional Application Number 61/495,843 titled "Methods and Apparatus for Estimating a Blood Volume of a Mammalian Subject" filed on June 10, 2011, to U.S. Provisional Application Number 61,493,847 titled "Methods and Apparatus for Estimating a Blood Volume of a Mammalian Subject" filed on June 6, 2011, and to U.S. Provisional Application Number 61/365,270 titled "Methods and Apparatus for Estimating a Blood Volume of a Mammalian Subject" filed on July 16, 2010.

### TECHNICAL FIELD

Embodiments of the present invention relate generally to blood volume estimation, absolute or relative, and changes in blood volume, and more particularly to blood volume estimation using heart rate, electrical body impedance, temperature, blood flow, and/or humidity measurements.

### BACKGROUND

There are a variety of situations where it is useful to measure a blood volume of a mammalian subject. For example, it can be useful to measure a blood volume of a subject pre-, peri-, or post-operatively. It can also be useful to measure a blood volume of a subject in the case of certain known or suspected injuries, or in the case of certain diseases or disorders. By measuring blood volume at different points in time, and then comparing the measurements, it can be determined whether a subject is experiencing blood loss (e.g., bleeding) or blood gain (e.g., unnecessary transfusions).

In some cases, a blood volume of a subject may be measured in terms of the subject's central blood volume (i.e., the volume of blood in the subject's heart, lungs and central arterial tree). In other cases, a blood volume of a subject may be measured in terms of the volume of blood in the subject's circulatory system as a whole, or in terms of a portion of the subject's circulatory system that includes the subject's heart, pulmonary vessels and central arterial tree.

A common way of measuring blood volume relies on measurements taken using a Swan-Ganz catheter. However, these measurements are invasive, and they expose a subject to significant risks (e.g., potential rupture of the catheter's balloon, arrhythmia, pulmonary infarction, thrombosis, and infection, to name a few).

### SUMMARY

A method for estimating a blood volume of a mammalian subject according to embodiments of the present invention includes, using an electronic apparatus, computing a blood volume index from hemodynamic data for the subject, the blood volume index defined in terms of a baseline electrical body impedance of the subject, a baseline heart rate of the subject, an electrical body impedance of the subject at a time of interest, and a heart rate of the subject at the time of interest, and using the blood volume index to generate an output indicative of an estimated blood volume of the subject. The output indicative of the estimated blood volume of the subject may be an output indicative of an estimated relative blood volume of the subject, and using the blood volume index to generate the output indicative of the estimated blood volume may include mapping the blood volume index to one of a ratio or a display value. Such mapping may be performed using one of a formula, model, table or data structure derived from relationships between blood volume indices and relative blood volumes of subjects other than the subject for which a blood volume is being estimated.

The output indicative of the estimated blood volume of the subject may be an output indicative of an estimated actual blood volume of the subject, and using the blood volume index to generate the output indicative of the estimated blood volume may include i) mapping the blood volume index to a ratio, and ii) multiplying a baseline blood volume for the subject by the ratio. Such mapping may be performed using one of a formula, model, table or data structure derived from relationships between blood volume indices and relative blood volumes of subjects other than the subject for which a blood volume is being estimated. The output indicative of the estimated blood volume may be an output indicative of an estimated central blood volume. According to embodiments of the present invention, the blood volume index is defined in terms of a relative heart rate and a relative electrical body impedance, the relative heart rate being a ratio of the heart rate of the subject at the time of interest over the baseline heart rate of the subject, and the relative electrical body impedance being a ratio of the electrical body impedance of the subject at the time of interest over the baseline electrical body impedance of the subject.

According to embodiments of the present invention, the blood volume index is defined in terms of (heart rate of the subject at the time of interest / baseline heart rate of the subject) * (1 + x*((electrical body impedance of the subject at the time of interest / baseline electrical body impedance of the subject) - 1)); where x is a multiplier other than one.

According to some embodiments, x is equal to or substantially the same as or approximately five.

According to embodiments of the present invention, the blood volume index is defined in terms of (heart rate of the subject at the time of interest / baseline heart rate of the subject) * (electrical body impedance of the subject at the time of interest / baseline electrical body impedance of the subject).

According to embodiments of the present invention, the blood volume index is defined in terms of 1 - ((heart rate of the subject at the time of interest - baseline heart rate of the subject) / baseline heart rate of the subject + ((change in electrical body impedance of the subject at the time of interest - baseline electrical body impedance) / baseline electrical body impedance of the subject)*5) / 6, wherein one or both of the baseline heart rate and baseline electrical body impedance are calculated as averages of a plurality of measurements taken over a period of time.

The electronic apparatus may be used to acquire hemodynamic measurements from the subject, and process the hemodynamic measurements to generate the hemodynamic data. According to embodiments of the present invention, the baseline electrical body impedance and the electrical body impedance at the time of interest are thoracic or thigh impedances.

An apparatus for estimating a blood volume of a mammalian subject according to embodiments of the present invention includes a number of cardiovascular signal sensors, communicable with the subject to obtain hemodynamic measurements from the subject, and at least one integrated circuit, operably connected with the number of cardiovascular signal sensors and configured to i) compute a blood volume index from the hemodynamic measurements, the blood volume index defined in terms of a baseline electrical body impedance of the subject, a baseline heart rate of the subject, an electrical body impedance of the subject at a time of interest, and a heart rate of the subject at the time of interest, and ii) use the blood volume index to generate an output indicative of an estimated blood volume of the subject.

An apparatus for estimating a blood volume of a mammalian subject according to embodiments of the present invention may include at least one integrated circuit configured to i) compute a blood volume index from hemodynamic data for the subject, the blood volume index defined in terms of a baseline electrical body impedance of the subject, a baseline heart rate of the subject, an electrical body impedance of the subject at a time of interest, and a heart rate of the subject at the time of interest, and ii) use the blood volume index to generate an output indicative of an estimated blood volume of the subject, as well as a perceptible output interface, operably connected with the at least one integrated circuit to receive the output indicative of the estimated blood volume, and operably connectable to a perceptible blood volume indicator to cause the perceptible blood volume indicator to convey an estimated blood volume of the subject to a user of the apparatus.

An apparatus for estimating a blood volume of a mammalian subject according to embodiments of the present invention may include a hemodynamic data input interface, an estimated blood volume output interface, a computer data storage having a stored set of machine-readable instructions, the machine-readable instructions defining a program executable by at least one processing unit, the machine-readable instructions including i) instructions defining a computation of a blood volume index from hemodynamic data, the blood volume index defined in terms of a baseline electrical body impedance of a subject, a baseline heart rate of the subject, an electrical body impedance of the subject at a time of interest, and a heart rate of the subject at the time of interest, and ii) instructions defining use of the blood volume index to generate an output indicative of an estimated blood volume of the subject, and at least one processing unit, operably connected with the hemodynamic data input interface, operably connected in program reading communication with the computer data storage, and operably connected with the estimated blood volume output interface.

A non-transitory computer readable medium according to embodiments of the present invention may have a stored set of machine-readable instructions, the machine-readable instructions executable by at least one processing unit, the machine-readable instructions defining a computer program for estimating a blood volume of a mammalian subject, the machine-readable instructions including instructions that, when executed by the at least one processing unit, cause the at least one processing unit to compute a blood volume index from hemodynamic data for the subject, the blood volume index defined in terms of a baseline electrical body impedance of the subject, a baseline heart rate of the subject, an electrical body impedance of the subject at a time of interest, and a heart rate of the subject at the time of interest, and use the blood volume index to generate an output indicative of an estimated blood volume of the subject.

A method for detecting a circulatory disorder, such as deep vein thrombosis, in a mammalian subject according to embodiments of the present invention includes, using an electronic apparatus, computing a blood volume index from hemodynamic data for the subject over time, the blood volume index defined in terms of a baseline electrical body impedance at an extremity of the subject, a baseline heart rate of the subject, an electrical body impedance at the extremity of the subject at a time of interest, and a heart rate of the subject at the time of interest, identifying changes in the blood volume index over time, and correlating changes in the blood volume index over time at the extremity as indicative of a circulatory disorder.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

The invention is defined by the appended claims, the description with examples and embodiments serving only for illustrative purposes.

BRIEF DESCRIPTION OF THE DRAWINGS

Illustrative embodiments of the invention are illustrated in the drawings, in which:

FIGS. 1A-1I illustrate corresponding hemodynamic parameters, relative parameter values, and blood volume indices for nine human subjects;
FIGS. 2A-2I provide graphs of relative parameter values versus relative central blood volumes and are based on the relative parameter values shown in respective ones of FIGS. 1A-1I;
FIGS. 3A-3I provide graphs of blood volume indices versus relative central blood volumes and are based on the blood volume indices and relative central blood volumes shown in respective ones of FIGS. 1A-11;
FIG. 4 is a composite graph of the blood volume indices labeled BVI #2 in FIGS. 1A-1I;
FIG. 5 illustrates an exemplary method for estimating a blood volume of a mammalian subject;
FIG. 6 illustrates a first exemplary apparatus for estimating the blood volume of a subject;
FIG. 7 illustrates a second exemplary apparatus for estimating the blood volume of a subject;
FIG. 8 illustrates a block diagram of exemplary structural components that can be used to implement an apparatus for estimating blood volume;
FIG. 9 illustrates a block diagram of an exemplary non-transitory computer readable medium having a stored set of machine-readable instructions;
FIG. 10 illustrates an exemplary band to which ECG-type electrodes are coupled; and
FIG. 11 illustrates an exemplary graphical display that can be displayed on a computer display screen of a blood volume monitor.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

DETAILED DESCRIPTION

Studies have shown that changes in electrical body impedance are indicative of changes in various hemodynamic parameters, such as changes in heart rate, cardiac input/output, fluid levels, and mean arterial pressure. "Electrical body impedance" is a measurement of the resistance, at a particular voltage, to electrical current flow through part of a subject's body. Measurements of electrical body impedance can be taken for a subject's body as a whole, or for various parts of (or paths through) the subject's body. One common measurement of electrical body impedance is "thoracic impedance", which is the impedance across a subject's thoracic region. Other electrical body impedance measurements include, for example, "whole body impedance" (which, in humans, is typically the impedance between the hand on one side of the person's body and the foot on the other side of the person's body), or the impedance across an extremity, such as across a subject's thigh, ankle, knee, wrist, bicep, or elbow.

Conventional electrical body impedance often focuses on thoracic impedance measurements. For purposes of measuring electric body impedance as a part of determining blood volume and particularly changes in blood volume, measuring electrical body impedance at extremities may provide a more accurate or quicker blood volume related measurement as compared to thoracic measurements. When a body is under stress, such as from bleeding, it often shunts blood from the extremities to the thoracic and head region to protect organs and the brain. Moreover, in the case of internal bleeding, it can be the case that blood pools in the chest cavity or abdominal region. In both cases, while blood loss is occurring, the blood volume in the thoracic area may not be changing sufficiently for detection. Moreover, in both cases, blood volume is decreasing more dramatically in the extremities.

Besides measuring electrical impedance at extremities, it is also possible to measure relative differences in electrical impedance between the thoracic area and an extremity. With such a comparison, the system may be able to detect internal bleeding. With internal bleeding, changes in electrical impedance in the thoracic region may change slowly or not all whereas changes in electrical impedance at an extremity may change more quickly or change when the thoracic impedance does not change. In such situations, the system may present a prompt, alarm, digital read out, or other indication that internal bleeding is possible.

The use of electrical body impedance to measure (or monitor) changes in hemodynamic parameters is attractive because electrical body impedance can be measured in various safe and non-invasive ways. For example, electrical body impedance can be measured using an electrocardiograph or electrocardiography (ECG) techniques and bioelectrical impedance analysis systems. Based on the present disclosure, one of ordinary skill in the art will appreciate various other ways in which electrical body impedance can be measured.

It is known that changes in blood volume can cause changes in electrical body impedance. However, given that electrical body impedance is affected by so many other parameters, including other hemodynamic parameters, it is difficult to use electrical body impedance, alone, as a good indicator of blood volume. Also, the nature of the relationship between blood volume and electrical body impedance can vary from subject to subject (e.g., person to person). For example, when a person is bleeding, the ability or tendency of the body to shunt blood to the organs and brain often varies from person to person.

When a subject experiences blood loss due to external or internal bleeding (e.g., bleeding in the abdominal cavity), and the lost blood is not replaced with enough blood or fluid to ensure a fluid balance, the subject's heart experiences stress, which may lead to loss of consciousness and sometimes even death. Because of this stress, it was hypothesized that either heart rate, mean arterial pressure or both would increase to compensate for such stress. If the relationship between blood volume and one or both of heart rate and mean arterial pressure could be documented, it was hypothesized that a combination of electrical body impedance and one of these other parameters could be used as a more reliable indicator of blood volume.

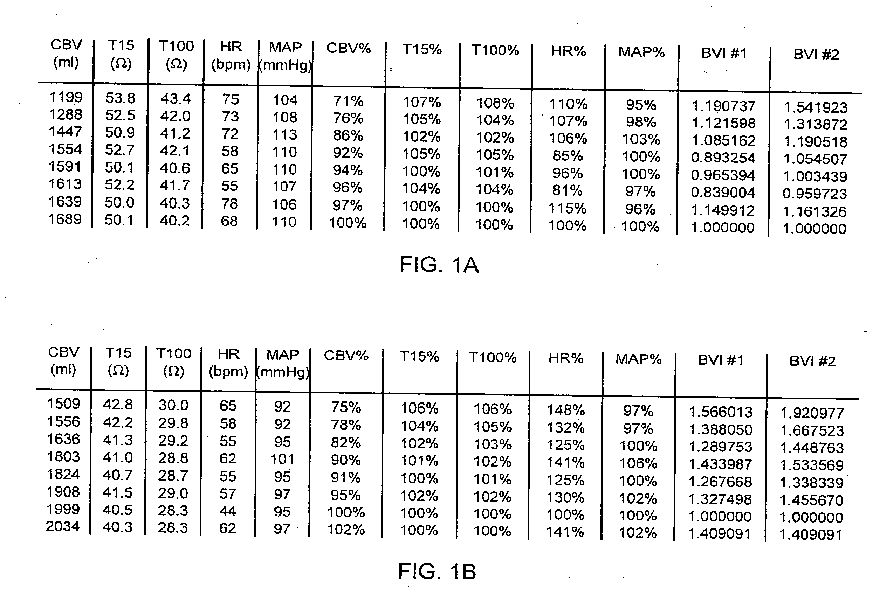

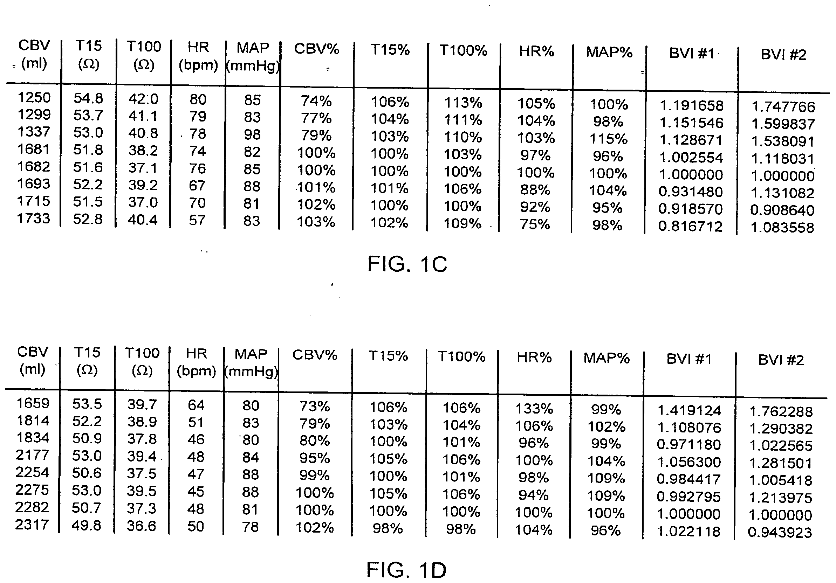

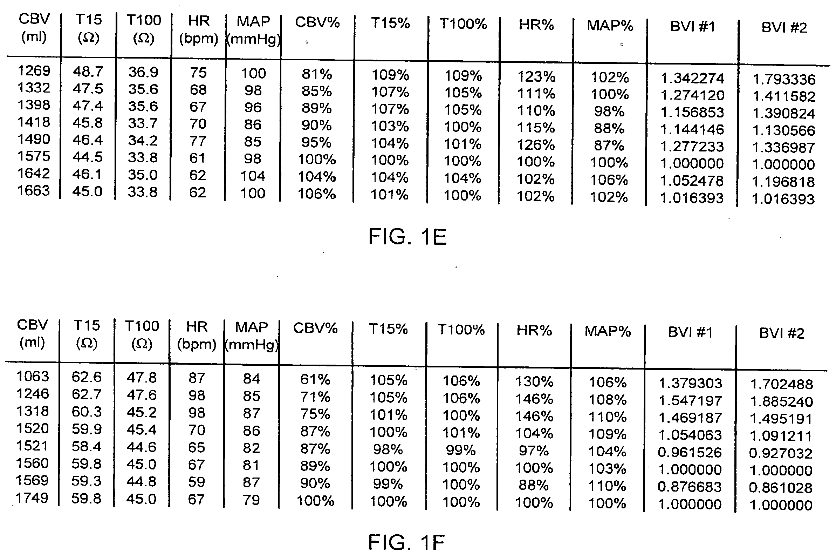

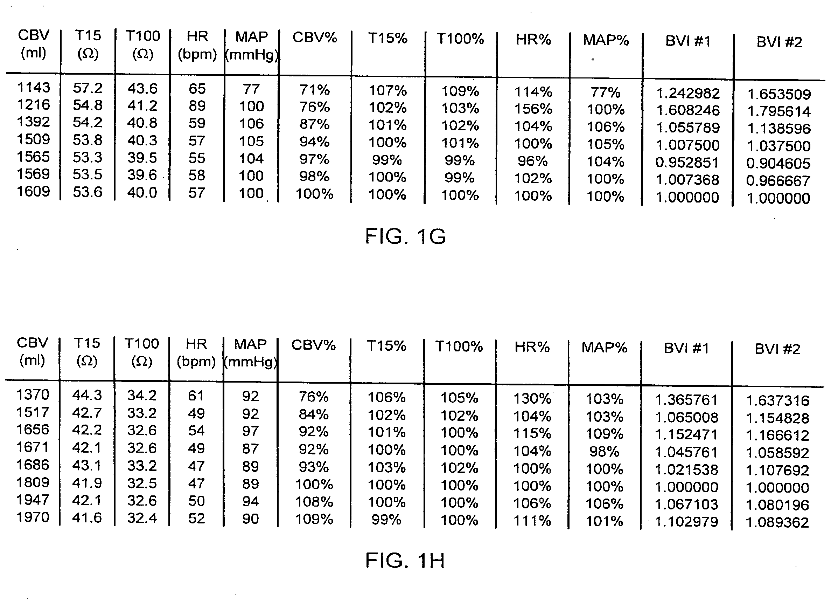

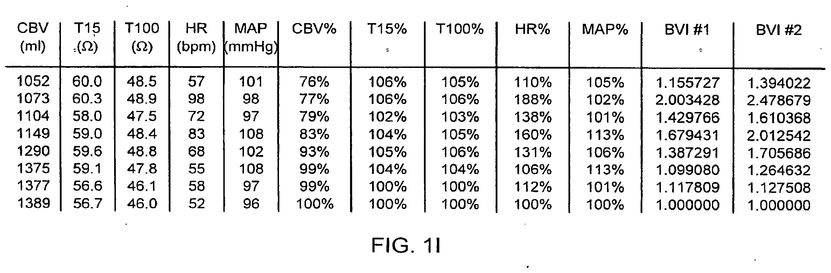

In assessing the above hypothesis, the results of a prior clinical study were examined. In the study, nine human subjects underwent clinical procedures during which blood was withdrawn from and added to their circulatory systems. The relative central blood volume (CBV), thoracic impedance (TI), heart rate (HR) and mean arterial pressure (MAP) of each subject were measured at various times before, during and after their procedure. In particular, the thoracic impedance was measured by applying a 200 microamp (200 $\mu$A) sine wave across a first pair of ECG electrodes while measuring a voltage across a second pair of ECG electrodes. One electrode of each pair was positioned on the subject's neck, and the other electrode of each pair was positioned on the subject's torso, under their arm. A first TI reading (T15) was acquired while applying a 1.5 kilohertz (1.5 kHz) sine wave across the first pair of electrodes, and a second TI reading (T100) was acquired while applying a 100 kHz sine wave across the first pair of electrodes. Stimulation frequencies falling in the range between 30 kHz and 100 kHz are possible. Corresponding CBV, T15, T100, HR and MAP parameters for the nine subjects (Subjects #1 - #9) are shown in FIGS. 1A-1I.

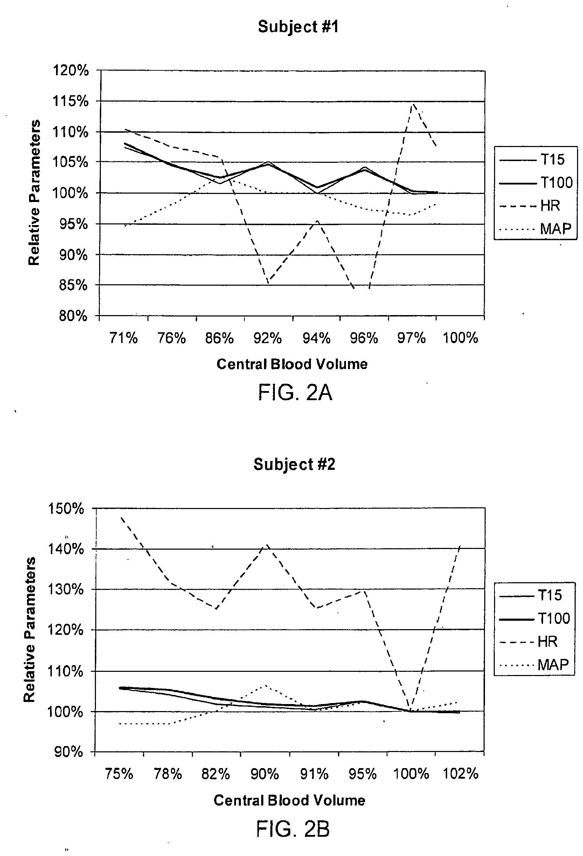

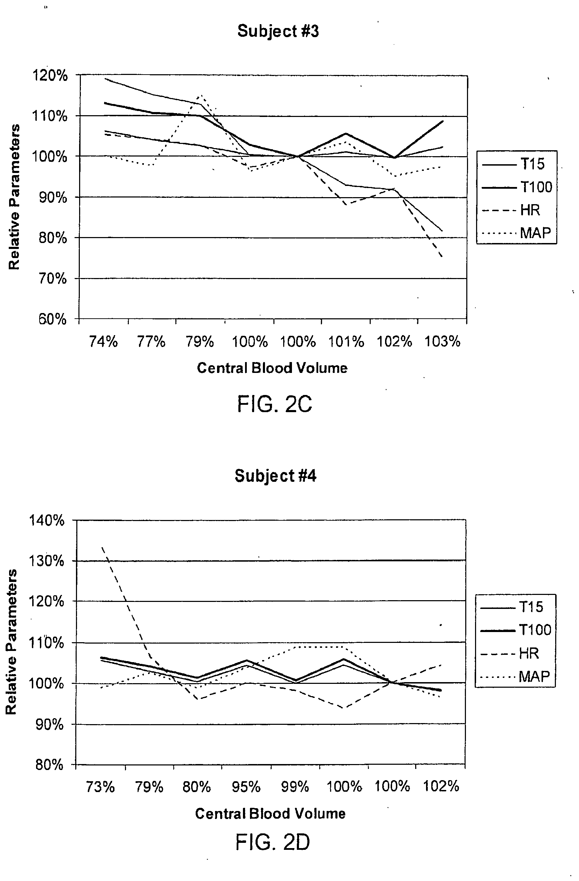

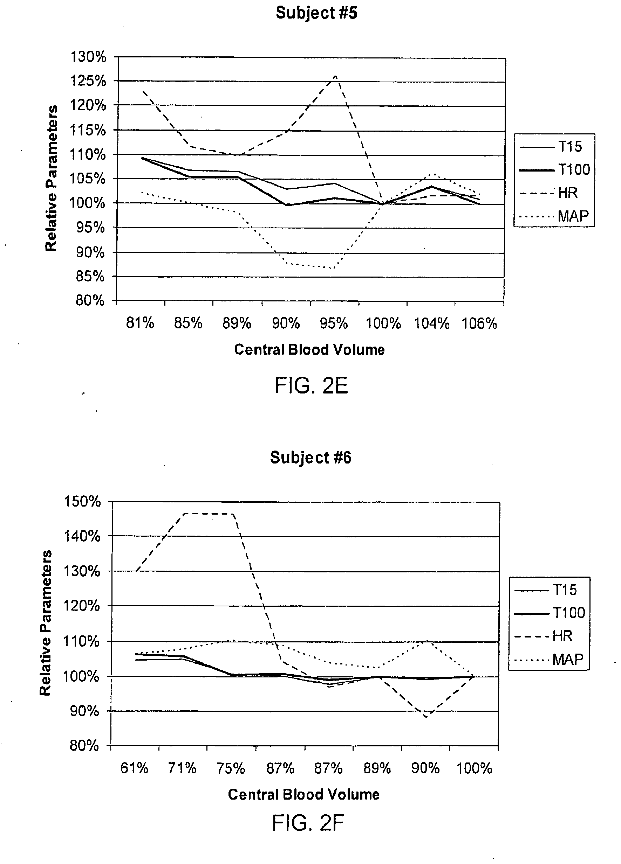

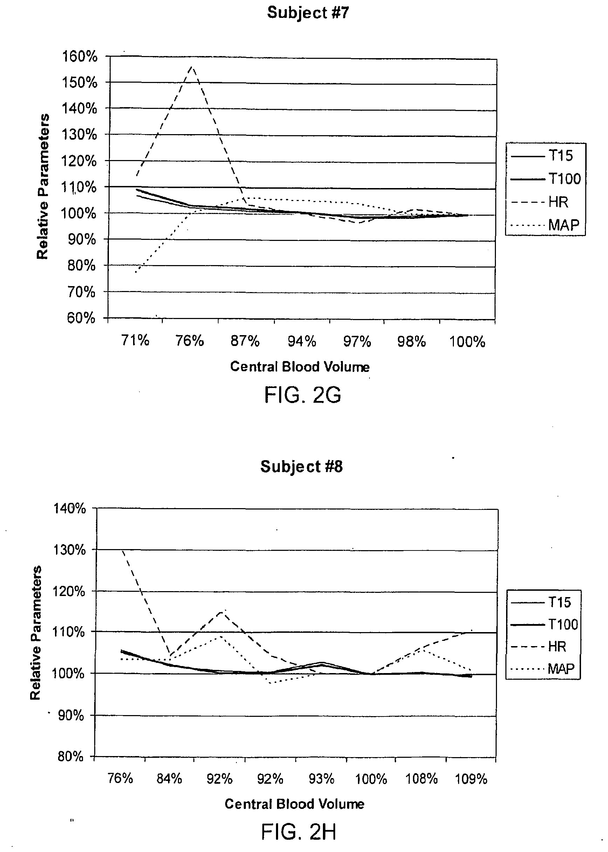

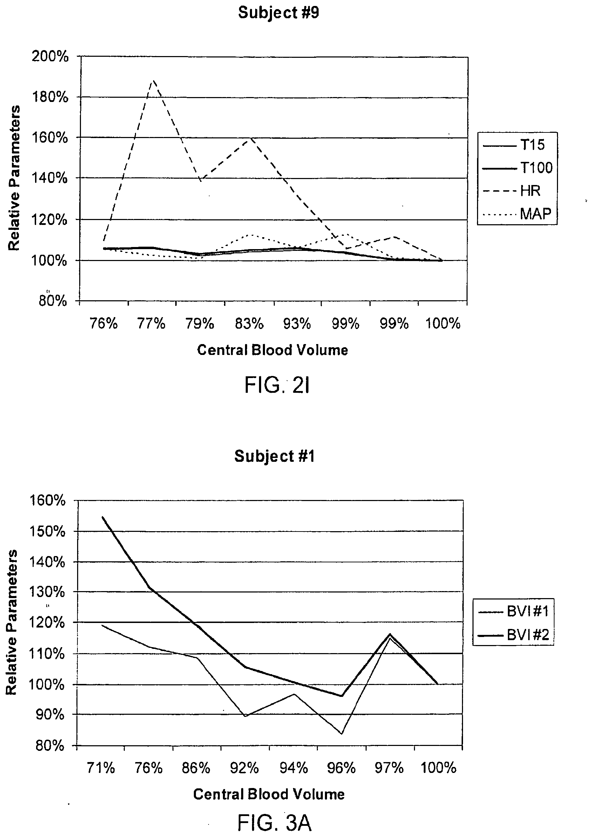

Given the high degree of variation in the parameters obtained for the different subjects, relative parameter values are derived by dividing each parameter by a respective baseline parameter. In some implementations, the baseline parameters for each subject corresponded to a central blood volume that is believed to be normal (i.e., 100% CBV) for the subject. The relative parameter values for each subject are shown in FIGS. 1A-1I as CBV%, T15%, T100%, HR% and MAP%. T15%, T100%, HR% and MAP% are plotted versus CBV% in FIGS. 2A-2I. In other implementations, the baseline parameters correspond to a starting point, such as a particular point in time when the parameters are measured, and the relative parameter values reflect changes in comparison to the baseline parameters. Thus, for example, in some implementations changes in blood volume are relative to a baseline reflective of the normal blood volume whereas in other implementations changes in blood volume are not necessarily relative a baseline blood volume that is reflective of the normal blood volume.

From the plots of the relative parameter values, or through statistical analysis, it can be determined that both thoracic impedance (both at T100 and T15) and heart rate bear a relationship to relative central blood volume. However, none of the relationships between T15%, T100%, HR% and CBV% are strong enough that thoracic impedance or heart rate, taken alone, can be used as a reliable indicator of central blood volume. Mean arterial pressure does not appear to have any relationship to relative central blood volume.

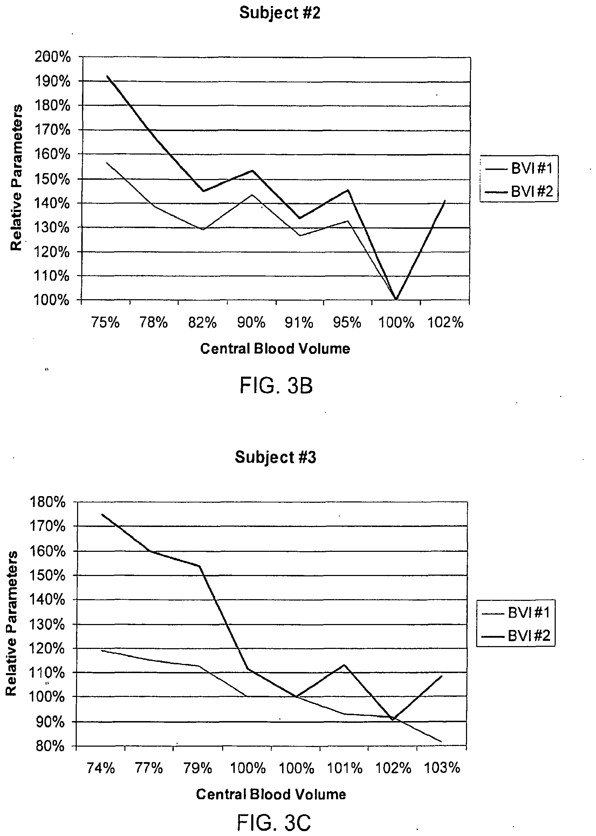

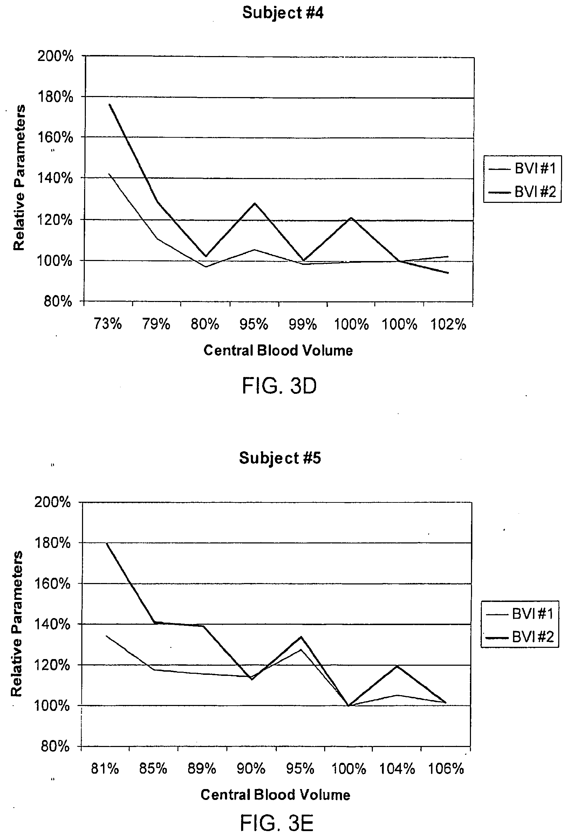

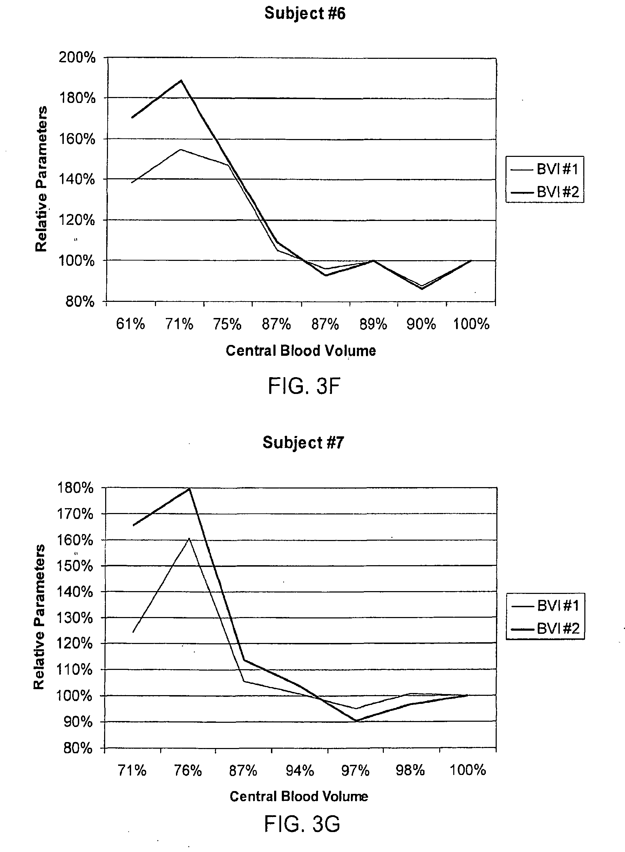

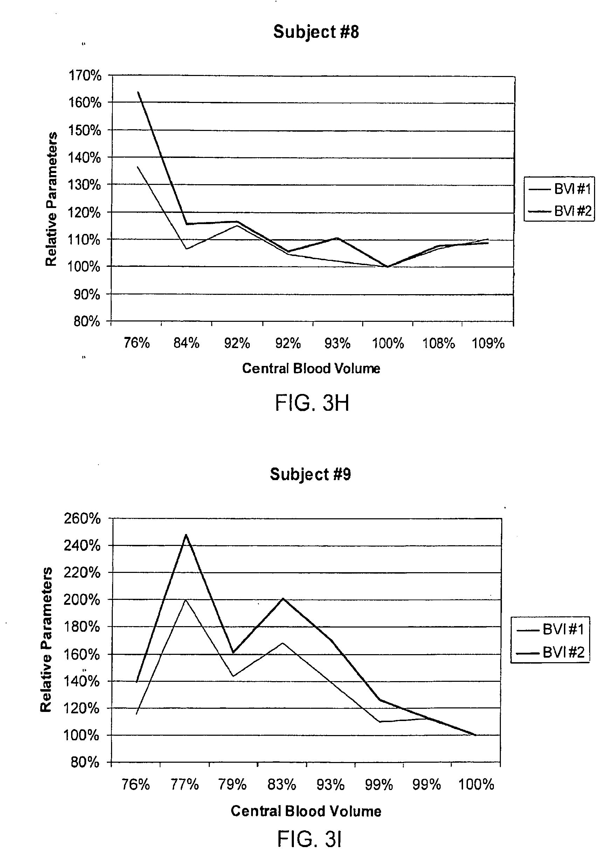

As previously indicated, it was desired to determine whether electrical body impedance, when combined with one or more other measurements, might serve as a reliable indicator of central blood volume. In this regard, corresponding relative heart rates (HR%) and relative thoracic impedances (T100%) were multiplied to produce a first set of blood volume indices for each subject. These blood volume indices, denoted BVI #1, are shown in FIGS. 1A-1I and are plotted versus relative central blood volume for each subject in FIGS. 3A-3I.

As can be seen, for most subjects there is a fairly strong correlation between BVI #1 and relative central blood volume. For example, referring to Fig. 3A, BVI #1 rises from 90% to 120% as central blood volume decreases from 92% to 71%. In another example, referring to Fig. 3G, BVI #1 rises from about 96% to 160% as central blood volume decreases from 97% to 76%. Numerous other examples are shown in Figs. 3A-3I.

In addition to simple multiplication of HR% and T100%, corresponding relative heart rates and relative thoracic impedances may be combined in other ways. For example, relative heart rates and relative thoracic impedances can be combined according to the following formula:

$$BVI = HR\% * (1 + x*(T100\% - 1)); \qquad\qquad (1)$$

where x is a multiplier other than one.

Applying formula (1) to the relative parameter values provided in FIGS. 1A-1I, with x = 5, yields a second set of blood volume indices for each subject. These second sets of blood volume indices, denoted BVI #2, are shown in FIGS. 1A-1I and are plotted versus relative central blood volume for each subject in graphs FIGS. 3A-3I. As one can see, there is an even stronger correlation between BVI #2 and relative central blood volume.

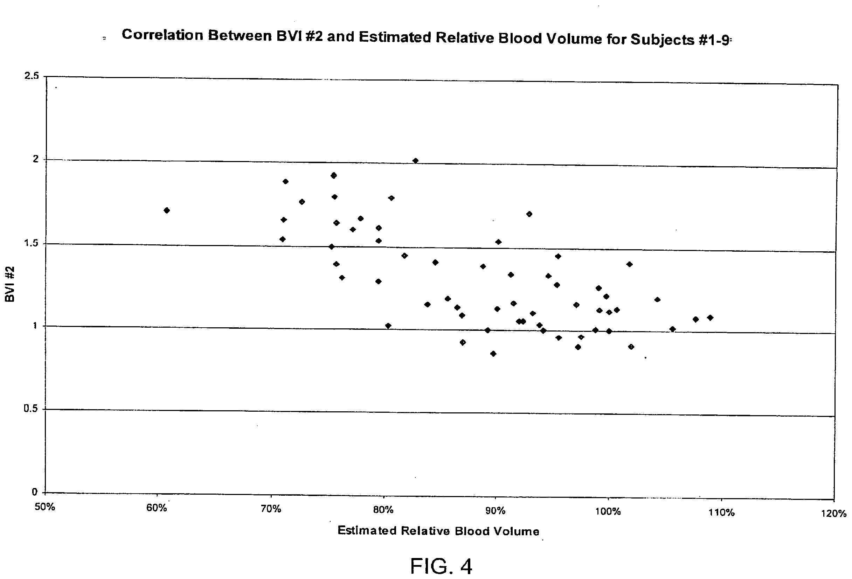

FIG. 4 is a composite graph of the second sets of blood volume indices for all nine subjects (i.e., a graph of BVI #2 values). One can see that the values of the blood volume indices generally rise higher as relative central blood volume decreases, and conversely, fall lower as relative central blood volume increases. Thus, blood volume indices based on a combination of electrical body impedance and heart rate can be used to estimate the relative central blood volume of a mammalian subject (and more particularly, a human subject).

Other combinations of HR% and T100% include combining changes in relative heart rates with changes in relative thoracic impedances according to the following formula:

$$\text{BVI} = 1 - \Delta\text{HR\%} + (\Delta\text{T100\%} * 5) / 6. \tag{2}$$

Applying formula (2) to the relative parameter values provided in Table 1, where the baseline heart rate and baseline thoracic impedance are an average of 5 readings over time, yields a third set of blood volume indices, denoted BVI #3, for each subject. Formula (2) can be similarly applied to changes in T15% to calculate BVI #3. It should be noted, as discussed herein, while BVI #1 - BVI #3 reference a thoracic impedance value, other extremity impedance measurements, some of which are mentioned herein, may be used in place of or in conjunction with thoracic impedance measurements.

Where baseline heart rate and/or baseline electrical body impedance are average readings, baseline heart rate and baseline electrical body impedance may be calculated as an average using any appropriate number of measurements over any appropriate time frame or interval. For example, baselines may be calculated, without limitation, as an average of 5-15 measurements over 50-150 seconds, 2-10 measurements over 18-80 seconds, 3-5 measurements over 30-45 seconds, or the like.

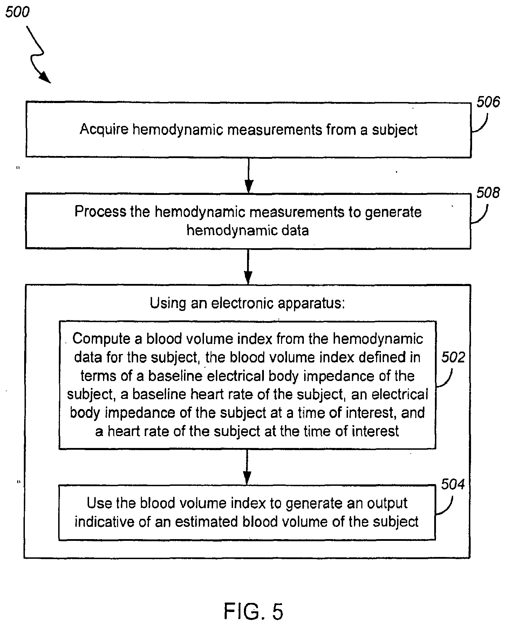

In light of the conclusions drawn from the above-described study, FIG. 5 illustrates an exemplary method 500 for estimating a blood volume of a mammalian subject. At block 502, the method uses an electronic apparatus to compute a blood volume index from hemodynamic data for the subject. The electronic apparatus may comprise, for example, one or more integrated circuits defining: a processing unit, a microcontroller, a microprocessor, an application specific integrated circuit (ASIC), or a programmable gate array. The blood volume index is defined in terms of a baseline electrical body impedance (EBI) of the subject, a baseline heart rate of the subject, an electrical body impedance of the subject at a time of interest, and a heart rate of the subject at the time of interest. Using the electronic apparatus, the blood volume index is used to generate an output indicative of an estimated blood volume of the subject (at block 504). By way of example, the output may take the form of 1) a value that is written to a non-transitory computer readable medium (e.g., a random access memory (RAM), one or more data storage registers, a hard disk, or a removable disk) for later use, or 2) a value or instruction that causes the estimated blood volume to be displayed in real-time via a perceptible blood volume indicator, such as a computer display screen or printer.

Optionally, the method 500 may include 1) acquiring hemodynamic measurements from the subject (at block 506), and 2) processing the hemodynamic measurements to generate the hemodynamic data that is used to compute the blood volume index (at block 508). By way of example, the hemodynamic measurements may take the form of measured voltages, waveforms or pulse counts. The generation of hemodynamic data from such measurements may comprise, for example, computing impedance values or heart rates from the hemodynamic measurements.

The blood volume index (BVI) calculated by the method 500 may take various forms, including, for example:

$$\text{BVI \#1} = \text{(heart rate of the subject at the time of} \tag{3}$$

interest / baseline heart rate of the subject)

* (electrical body impedance of the subject at the time of interest / baseline electrical body impedance of the subject); or

$$BVI\ \#2 = \text{(heart rate of the subject at the time of interest} \tag{4}$$
$$/ \text{ baseline heart rate of the subject)} * (1 +$$
$$x*((\text{electrical body impedance of the subject at}$$
$$\text{the time of interest / baseline electrical body}$$
$$\text{impedance of the subject)} - 1));$$

where x is a multiplier other than one; or

$$BVI\ \#3 = 1 - ((\text{heart rate of the subject at the time of} \tag{5}$$
$$\text{interest - baseline heart rate of the subject})$$
$$/ \text{ baseline heart rate of the subject} + ((\text{change in}$$
$$\text{electrical body impedance of the subject at}$$
$$\text{the time of interest} - \text{baseline electrical body}$$
$$\text{impedance)} / \text{ baseline electrical body}$$
$$\text{impedance of the subject)}*x) / x+1,$$

where the baseline is an average of a plurality of measurements taken over a period of time, where x is a multiplier other than one. x may be the number of readings used for the average or another integer value.

Defining the following relative parameter values:

$$HR\% = \text{heart rate of the subject at the time of interest} \tag{6}$$
$$/ \text{ baseline heart rate of the subject;}$$

$$EBI\% = \text{electrical body impedance of the subject at} \tag{7}$$
$$\text{the time of interest} / \text{ baseline electrical body}$$
$$\text{impedance of the subject;}$$

$$\Delta HR\% = \text{(heart rate of the subject at the time of interest} \tag{8}$$
$$\text{- baseline heart rate of the subject)} /$$
$$\text{baseline heart rate of the subject; and}$$

$$\Delta BVI\% = \text{(electrical body impedance of the subject} \tag{9}$$
$$\text{at the time of interest} - \text{baseline electrical}$$
$$\text{impedance of the subject)} / \text{ baseline electrical}$$
$$\text{impedance of the subject;}$$

the preceding blood volume indices may be rewritten as:

$$BVI\ \#1 = \quad HR\% * EBI\%; \tag{10}$$

$$BVI\ \#2 = \quad HR\% * (1 + x*(EBI\% - 1)); \tag{11}$$

where x is a multiplier other than one; and

$$BVI\ \#3 = \quad 1 - (\Delta HR\% + \Delta EBI\% * x) / x+1 \tag{12}$$

where x is a multiplier other than one.

In some embodiments, the blood volume index described by Equations (4), 5,(11), and (12) may be computed with x = 5.

In some embodiments, BVI #3 is calculated after first recording several HR and EBI measurements over several seconds, and calculating the baseline HR and EBI as an average over a portion of those measurements. For example, BVI #3 can be calculated after first recording 10 HR and EBI measurements each spaced by 10 second intervals, then calculating the baseline HR and EBI as an average of the last 5 HR and EBI measurements (a rolling HR measurement). Average HR may be calculated in other ways, as well, with more or less sequential readings being used for an average, measurements exceeding some threshold (e.g., 10% or 20% from the last reading) being discarded, and the like. Table 1, below, provides an example of data for calculating BVI #3 and the corresponding BVI #3 values. The example provided in Table 1 shows the baseline HR and EBI as being calculated after the first 10 readings from the average HR and EBI over measurements 6-10. $\Delta HR\%$ and $\Delta EB1\%$ are then calculated using Equations 8 and 9, respectively, and are shown in the respective columns of Table 1. Finally, BVI #3 is calculated using Formula 12, and is shown in the column labeled BVI #3.

Table 1: Data and Results for BVI #3. Measurements marked with an asterisk indicate possible artifacts and are not included in the calculations of the average EBI and HR.

|  | Time | EBI | HR | Average EBI | Average HR | $\Delta$EBI% | $\Delta$HR% | BVI #3 |
|---|---|---|---|---|---|---|---|---|
| 1 | 0:00:10 | 1,000 | 85 |  |  |  |  |  |
| 2 | 0:00:20 | 1,020 | 72 |  |  |  |  |  |
| 3 | 0:00:30 | 12,800* | 72 |  |  |  |  |  |
| 4 | 0:00:40 | 1,000 | 90* |  |  |  |  |  |
| 5 | 0:00:50 | 1,010 | 75 | 1,008 | 76 |  |  |  |
| 6 | 0:01:00 | 1,020 | 74 | 1,013 | 73 |  |  |  |
| 7 | 0:01:10 | 1,030 | 73 | 1,015 | 74 |  |  |  |
| 8 | 0:01:20 | 1,040 | 72 | 1,020 | 74 |  |  |  |
| 9 | 0:01:30 | 1,050 | 71 | 1,030 | 73 |  |  |  |
| 10 | 0:01:40 | 1,060 | 70 | 1,040 | 72 | 1.0% | -1.4% | 0.994 |
| 11 | 0:01:50 | 1,070 | 70 | 1,050 | 71 | 1.9% | -2.5% | 0.988 |
| 12 | 0:02:00 | 1,075 | 71 | 1,059 | 71 | 2.8% | -3.0% | 0.982 |
| 13 | 0:02:10 | 1,080 | 69 | 1,067 | 70 | 3.6% | -3.8% | 0.976 |
| 14 | 0:02:20 | 1,085 | 70 | 1,074 | 70 | 4.3% | -4.1% | 0.971 |
| 15 | 0:02:30 | 1,090 | 70 | 1,080 | 70 | 4.9% | -4.1% | 0.966 |
| 16 | 0:02:40 | 1,095 | 69 | 1,085 | 70 | 5.3% | -4.4% | 0.963 |
| 17 | 0:02:50 | 1,100 | 69 | 1,090 | 69 | 5.8% | -4.9% | 0.960 |
| 18 | 0:03:00 | 1,060 | 70 | 1,086 | 70 | 5.4% | -4.7% | 0.962 |
| 19 | 0:03:10 | 1,040 | 70 | 1,077 | 70 | 4.6% | -4.7% | 0970 |
| 20 | 0:03:20 | 1,050 | 71 | 1,069 | 70 | 3.8% | -4.4% | 0.976 |
| 21 | 0:03:30 | 1,080 | 72 | 1,066 | 70 | 3.5% | -3.6% | 0.977 |
| 22 | 0:03:40 | 1,100 | 71 | 1,066 | 71 | 3.5% | -3.0% | 0.976 |
| 23 | 0:03:50 | 1,050 | 72 | 1,064 | 71 | 3.3% | -2.5% | 0.977 |

(continued)

| | | Time | EBI | HR | Average EBI | Average HR | ΔEBI% | ΔHR% | BVI #3 |
|---|---|---|---|---|---|---|---|---|---|
| | 24 | 0:04:00 | 1,080 | 73 | 1,072 | 72 | 4.1% | -1.6% | 0.969 |
| | 25 | 0:04:10 | 1,200 | 71 | 1,102 | 72 | 7.0% | -1.6% | 0.944 |
| | 26 | 0:04:20 | 1,210 | 74 | 1,128 | 72 | 9.5% | -1.1% | 0.923 |
| | 27 | 0:04:30 | 1,210 | 72 | 1,150 | 72 | 11.7% | -0.8% | 0.904 |
| | 28 | 0:04:40 | 1,220 | 75 | 1,184 | 73 | 15.0% | 0.0% | 0.875 |
| | 29 | 0:04:50 | 1,230 | 72 | 1,214 | 73 | 17.9% | -0.3% | 0.852 |
| | 30 | 0:05:00 | 1,200 | 75 | 1,214 | 74 | 17.9% | 0.8% | 0.850 |
| | 31 | 0:05:10 | 1,200 | 76 | 1,212 | 74 | 17.7% | 1.4% | 0.850 |
| | 32 | 0:05:20 | 1,200 | 73 | 1,210 | 74 | 17.5% | 1.6% | 0.852 |
| | 33 | 0:05:30 | 1,200 | 75 | 1,206 | 74 | 17.1% | 1.6% | 0.855 |
| | 34 | 0:05:40 | 1,200 | 75 | 1,200 | 75 | 16.5% | 2.5% | 0.858 |

In some embodiments, any of the equations and values identified as (1)-(12) are modified to incorporate changes in temperature and/or changes in the perfusion index as commonly measured in pulse-oximetry, peripheral blood flow (PBF) and skin humidity, to refine the BVI calculations. Generally, body temperature (T), as measured at an extremity (e.g., a non-thoracic site), is inversely correlated to BVI. Similarly, perfusion index (PI), as measured at an extremity, is inversely correlated to BVI. It is also possible to include a humidity measurement (H) (e.g., clamminess measurement) and further refine the blood volume indices. While temperature, for example, decreases when a body is under stress and losing blood volume, humidity tends to increase with blood volume decreases. Thus, for example, Equation (12) can be modified as shown in Equation (13), below.

$$BVI = (1 - (\Delta HR\% + \Delta EBI\% * x) / x+1) *(\Delta T*y) * (\Delta PBF*z) * (\Delta H*A) *(\Delta PI*B) \tag{13}$$

where x, y z, A and B are multipliers other than one. While various multipliers are illustrated herein as integer values, non-integer decimal values may also be possible.

Although the preceding exemplary blood volume indices are defined in terms of particular parameters (e.g., heart rate, electrical body impedance, body temperature, perfusion index, humidity, relative heart rate, relative electrical body impedance, relative body temperature, and relative perfusion index), these parameters need not be individually computed in advance of computing a blood volume index. That is, the blood volume indices described above need not be computed by first computing each of their component parameters or terms. Instead, the blood volume indices may be computed by combining and manipulating hemodynamic measurements and other values using algorithms that are more efficiently implemented by an electronic apparatus (e.g., by a programmed microcontroller). Despite these more efficiently implemented algorithms not computing each of the parameters or terms in the above equations on an individual basis, these more efficiently implemented algorithms are still considered to compute a blood volume index in accord with the above equations, so long as their outputs are equivalent to those that would have been generated by individually computing the parameters and terms identified in the above equations.

The output indicative of the estimated blood volume of a subject may be an output indicative of an estimated "relative" blood volume of the subject (i.e., a unitless value indicating a percentage or ratio of the subject's blood volume at a particular time, in relation to a baseline blood volume of the subject). A blood volume index may be used to generate an estimated relative blood volume by, for example, mapping the blood volume index to a ratio (or percentage). Alternately, a blood volume index may be mapped to a display value (such as a pixel value or chart position) that corresponds to a particular percentage or ratio indicator displayed on a computer display screen or printout. In either case, the mapping may be done, for example, using a formula or model that converts the blood volume index to the ratio or display value. Or, for example, the mapping may be done by using the blood volume index to look up a ratio or display value in a table or other data structure. Any such formula, model or table may be generated in advance of executing the method 500 (FIG. 5), and may be derived from clinical data such as the data provided in FIGS. 1A-1I. The clinical data may provide relationships between blood volume indices and relative blood volumes of subjects other than the subject for which a relative blood volume is being estimated. In some embodiments, different formulas, models or tables may be developed and selected based on a subject's physical characteristics, health, demographics, or other factors.

In other embodiments of the method 500, the output indicative of the estimated blood volume may be indicative of an estimated "actual" blood volume of the subject (i.e., an output associated with a volume unit, such as pints or liters). An estimated actual blood volume may be computed, for example, by 1) measuring or obtaining a baseline blood volume for a subject, 2) mapping the blood volume index to a ratio (as described in the preceding paragraph), and then 3) multiplying the baseline blood volume by the ratio.

By way of example, the estimated blood volume derived from the method 500 may be an estimated central blood volume, an estimated blood volume for a subject's circulatory system as a whole, or an estimated blood volume for a portion of the subject's circulatory system including the subject's heart, pulmonary vessels and central arterial tree. The nature of the estimated blood volumes depends, to some extent, on the nature of the electrical body impedances on which the estimated blood volume is based. For example, if the electrical body impedance is a thoracic impedance, the estimated blood volume will be an estimate of central blood volume. If the electrical body impedance is a thigh impedance, the estimated blood volume will be an estimate of the volume of blood in more than just the subject's heart, pulmonary vessels and central arterial tree; however, the estimated blood volume will typically have a significant correlation to the subject's central blood volume. It is noted that the method 500 may be based on various types of electrical body impedance, as might be useful for a particular application or purpose.

Having generally described a method for estimating the blood volume of a subject, estimating changes in blood volume, identifying parameters (used in conjuction) that are indicative of blood volume changes, and various exemplary systems and apparatus for implementing this and other methods are described below.

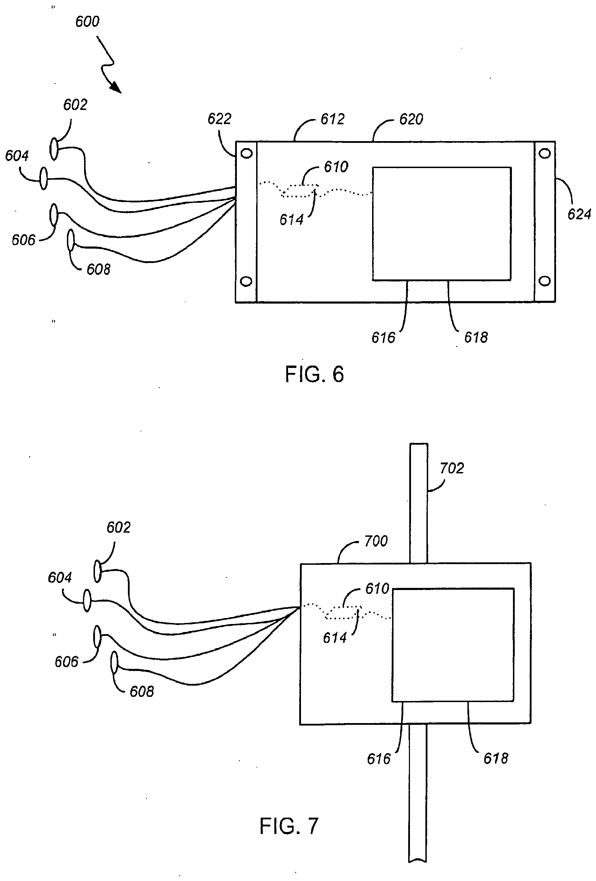

FIG. 6 illustrates a first exemplary apparatus 600 for estimating the blood volume of a subject. The apparatus comprises a number of cardiovascular signal sensors 602, 604, 606, 608 that are communicable with the subject to obtain hemodynamic measurements from the subject. The cardiovascular signal sensors may take various forms; according to some embodiments of the present invention, the sensors are non-invasive (or minimally invasive) types of sensors. For example, the sensors may take the form of (or comprise) ECG-type electrodes. Or, for example, the sensors might comprise a pulse oximeter (e.g., for acquiring a heart rate and/or blood oxygen content). The sensors may further include temperature sensors or other sensing devices used to determine a subject's body temperature, humidity sensors, and blood flow sensors.

The apparatus 600 further comprises at least one integrated circuit 610, operably connected with the number of cardiovascular signal sensors 602, 604, 606, 608. The at least one integrated circuit 610 is configured to compute a blood volume index from the hemodynamic measurements made by the cardiovascular signal sensors 602, 604, 606, 608. The blood volume index may be defined in terms of a baseline electrical body impedance of the subject, a baseline heart rate of the subject, an electrical body impedance of the subject at a time of interest, and a heart rate of the subject at the time of interest, and may take the forms previously described in equations (3), (4), (5), (10), (11), and (12), or other forms. Blood volume indices may further be defined in terms of body temperature, humidity and the skin, and blood flow. As previously mentioned, the fact that the blood volume index is defined in particular terms does not mean the apparatus 600 has to compute the blood volume index by individually computing these terms. Some measurement and BVI factors may be provided through sensors, through data inputs, manually entered, or otherwise.

The at least one integrated circuit 610 is further configured to use the blood volume index to generate an output indicative of an estimated blood volume of a subject. By way of example, the output may take the form of a value that is written to computer data storage for later use, and/or a value or instruction that causes an estimated blood volume to be displayed in real-time via a perceptible blood volume indicator, such as a computer display screen or printer. The output may take the form of a value that is written to or transmitted to another electronic device, according to embodiments of the present invention.

The at least one integrated circuit 610 may be provided as part of a stand-alone blood volume monitor 612, or as part of a multi-function device that monitors other parameters (e.g., blood pressure, blood oxygen, respiration, or the like) or performs other functions. For example, the at least one integrated circuit may be provided as part of an EKG monitor and/or a multifunction medical station. In some embodiments, the at least one integrated circuit is provided as part of a blood-pressure cuff readout, with signal sensors 602, 604, 606, 608 integrated within the blood-pressure cuff.

The at least one integrated circuit 610 may take various forms and may comprise, for example, a processing unit, a microcontroller, a microprocessor, an application specific integrated circuit (ASIC), or a programmable gate array. The at least one integrated circuit may also comprise computer data storage, such as a memory or hard disk. Alternately, the at least one integrated circuit may be connected to external computer data storage. The at least one integrated circuit may retrieve instructions or data from the computer data storage, and may also write data to the computer data storage. In some cases, the at least one integrated circuit 610 may be dedicated to blood volume monitoring, and possibly peripheral monitoring functions. In other cases, the at least one integrated circuit 610 may be used to perform other functions of a multi-function device.

The apparatus 600 further comprises a perceptible output interface 614 that is operably connected with the at least one integrated circuit 610 to receive the output indicative of the estimated blood volume. The perceptible output interface 614 is also operably connectable with (or connected to) a perceptible blood volume indicator 616 to cause the perceptible blood volume indicator 616 to convey an estimated blood volume of a subject to a user of the apparatus 600. In some embodiments, the perceptible blood volume indicator 616 may comprise a computer display screen 618 or printer. The perceptible blood volume indicator 616 may also (or alternatively) comprise an audible alarm circuit, a tactile alarm circuit (e.g., a vibrator circuit), or another form of indicator. In some cases, the output of the at least one integrated circuit 610 may be provided to multiple perceptible blood volume indicators via a single perceptible output interface 614. In other cases, the at least one integrated circuit 610 may comprise different perceptible output interfaces for driving different perceptible blood volume indicators.

One or both of the number of sensors 602, 604, 606, 608 and the perceptible blood volume indicator 616 may be integrated with the blood volume monitor 612, coupled to the blood volume monitor 612 by wires or cables, or coupled to the blood volume monitor 612 via one or more wireless communication channels. Similarly, one or both of the number of sensors 602, 604, 606, 608 and the perceptible blood volume indicator 616 may communicate with the blood volume monitor 612 (and the at least one integrated circuit 610) from a local or remote location. For example, the blood volume monitor 612 and perceptible blood volume indicator 616 may be located in the same room as a subject to which the cardiovascular signal sensors 602,604, 606, 608 are coupled. Alternately, the blood volume monitor 612 and/or the perceptible blood volume indicator 616 could be located remotely from the room where the subject resides.

By way of example, FIG. 6 illustrates the at least one integrated circuit 610 housed in a rack-mountable housing 620 having rack mounts 622 and 624. In some cases, the housing 620 may take the form of an aluminum housing. The computer display screen 618 may be mounted within the housing 620, as shown. Alternately, a computer display screen could be coupled to an interface connector that is mounted to the housing 620.

FIG. 7 illustrates the at least one integrated circuit 610 housed within a blood volume monitor 700 mounted to a pole 702. This pole-mounted blood volume monitor 700 may be more useful in a pre- or post- operative environment, or in a non-surgical setting, whereas the rack-mounted blood volume monitor 612 may be more useful in an operating room.

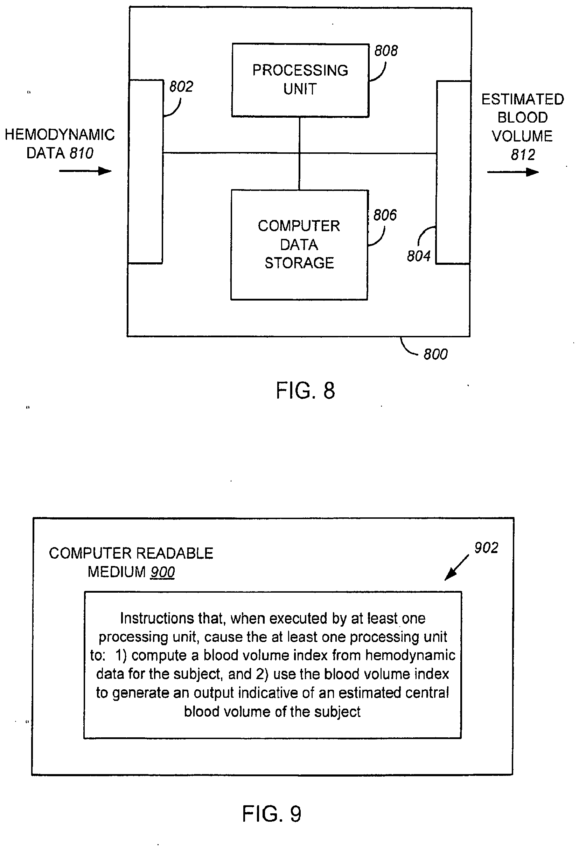

A blood volume monitor or multi-function device may be provided to a customer with or without signal sensors 602, 604, 606, 608. In this regard, FIG. 8 illustrates a block diagram of exemplary structural components 802, 804, 806, 808 that can be used to implement an apparatus 800 for estimating blood volume. The apparatus 800 comprises a hemodynamic data input interface 802, an estimated blood volume output interface 804, computer data storage 806, and at least one processing unit 808. In some embodiments, some or all of these elements 802, 804, 806, 808 may be provided by one or more integrated circuits.

The computer data storage 806 has a stored set of machine-readable instructions. The machine-readable instructions define a program that is executable by the at least one processing unit 808. The machine-readable instructions include i) instructions defining a computation of a blood volume index from hemodynamic data 810, and ii) instructions defining use of the blood volume index to generate an output 812 indicative of an estimated blood volume of the subject. According to some embodiments of the present disclosure, the blood volume index is defined in terms of a baseline electrical body impedance of a subject, a baseline heart rate of the subject, an electrical body impedance of the subject at a time of interest, and a heart rate of the subject at the time of interest.

The at least one processing unit 808 is operably connected with the hemodynamic data input interface 802 and the estimated blood volume output interface 804. The at least one processing unit 808 is also operably connected in program reading communication with the computer data storage 806. In operation, the at least one processing unit 808 reads and executes the program of machine-readable instructions. As the program is read and the instructions are executed, the at least one processing unit 808 receives or retrieves hemodynamic data 810 via the hemodynamic data input interface 802. The at least one processing unit 808 then uses the hemodynamic data 810 to compute the blood volume index and generate an output 812 indicative of a subject's estimated blood volume. This output 812 is provided to the estimated blood volume output interface 804.

In some embodiments of the apparatus 800, the hemodynamic data input interface 802 may be coupled to a number of cardiovascular signal sensors, or to external computer data storage from which the hemodynamic data 810 are retrieved. By way of example, the estimated blood volume output interface 804 may be coupled to a perceptible blood volume indicator, or to external computer data storage.

In some embodiments of the apparatus, hemodynamic data may be stored in the computer data storage 806. In these embodiments, the hemodynamic data input interface 802 may comprise some or all of the interface(s) that are used by the at least one processing unit 808 to read the program of machine-readable instructions from the computer data storage 806.

FIG. 9 illustrates a diagram of an exemplary non-transitory computer readable medium 900 having a stored set of machine-readable instructions 902. The machine-readable instructions 902 are executable by at least one processing unit and define a computer program for estimating a blood volume of a mammalian subject. The machine-readable instructions 902 comprise instructions that, when executed by the at least one processing unit, cause the at least one processing unit to: 1) compute a blood volume index from hemodynamic data for the subject, and 2) use the blood volume index to generate an output indicative of an estimated blood volume of the subject. The blood volume index is defined in terms of a baseline electrical body impedance of the subject, a baseline heart rate of the subject, an electrical body impedance of the subject at a time of interest, and a heart rate of the subject at the time of interest. Optionally, the instructions 902 may also cause the at least one processing unit to retrieve hemodynamic data for the subject. This retrieval may include a retrieval of hemodynamic measurements from a subject whose blood volume is being estimated, or a retrieval of measurements or other data from computer data storage. By way of example, the non-transitory computer readable medium 900 may comprise a random access memory (RAM), one or more data storage registers, a hard disk, and/or a removable disk.

Electrical body impedances and heart rates, and measurements on which these parameters are based, may be acquired in various ways. In some embodiments, measurements from which both electrical body impedance and heart rate can be derived are acquired using a set of four electrocardiograph type (ECG-type) electrodes (herein considered to be cardiovascular signal sensors), as shown in FIGS. 6 and 7. One pair of the electrodes may be positioned inside of the other pair of the electrodes with respect to a subject. A periodic signal may then be applied across the outer pair of electrodes. In some embodiments, the periodic signal may be a 100 kHz sine wave having a current amplitude of less than one milliamp (< 1 mA). As the current is applied, a voltage is measured across the second pair of electrodes, and impedances are derived from corresponding current and voltage measurements. A fast-moving (e.g., 50 millisecond (ms) sample rate) trend analysis of the impedances can be used to derive a heart rate, and a slow-moving (e.g., 15 second sample rate) trend analysis of the impedances can be used to derive an electrical body impedance that is largely independent of heart rate. Noise reduction, low-pass and notch-type filtering, as well as phase control, artifact neglect, and reliable electrode alarm monitoring, can all be applied during measurement acquisition. While impedances are primarily discussed herein, aspects of the present disclosure may also take advantage of or otherwise include resistance measurements, either alone or in conjunction with impedance measurements. If used alone, the resistance measurements would replace the impedance measurements in the formulations set out herein.

A processing unit may interpret acquired heart rate and electrical body impedance samples, and may use fuzzy logic or other signal processing to improve correlation between the samples.

In some embodiments, the ECG-type electrodes may be connected to leads that allow the electrodes to be independently positioned, In these embodiments, two of the electrodes may be positioned on a subject's neck, and two of the electrodes may be positioned on the subject's torso under their arm. The electrodes may also be positioned in other ways. The electrodes may take various forms, including the form of standard surface-mount ECG electrodes, such as electrodes that adhere to a subject via conductive gel and have snaps to which snap-on leads may be electrically connected.

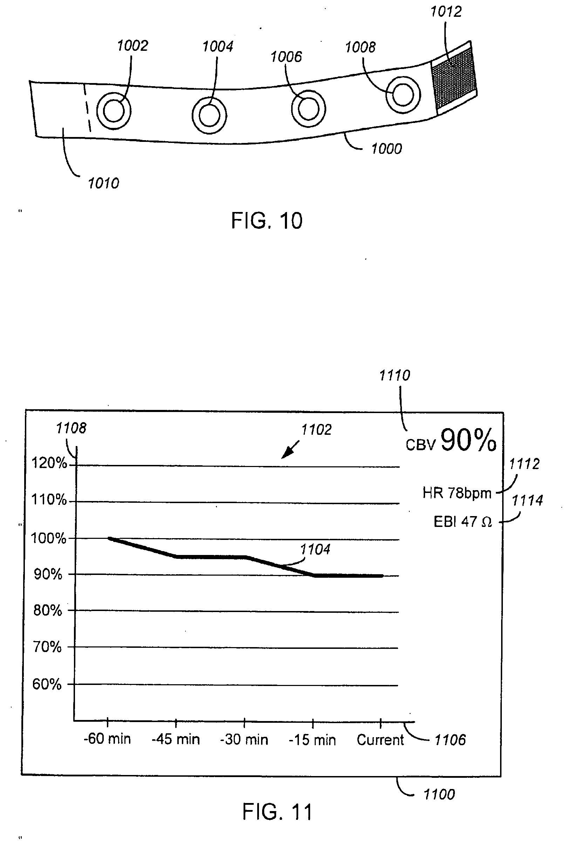

In other embodiments, the ECG-type electrodes 1002, 1004, 1006, 1008 may be coupled to a band 1000, such as an elastic, hook-and-loop 1010/1012 fastened band, as illustrated in FIG. 10. Such a band of electrodes is simple to position and can typically be reused many times. For a human subject, such a band 1000 may take the form of a thigh band having a plurality of electrodes 1002, 1004, 1006, 1008 disposed thereon. In use, the thigh band may be placed around one of the subject's thighs. In some embodiments, a band 1000 may take the form of an ankle band. In use, the ankle band may be placed around one of the subject's ankles.

Often, electrical body impedance changes due to blood loss are first noticed in a subject's extremities (e.g., a leg at, for example, an ankle, a knee, or a thigh, or an arm at, for example, a wrist, an elbow, or a bicep). In such cases where electrical body impedance is measured at an extremity impedance data may be collected relatively quickly, for example, measurements 1-5 times every second. In addition, changes in calculated blood volume at an extremity may provide indication of circulation disorders other than blood loss. For example, a rapid change in blood volume as calculated based on impedance measured at the ankle may indicate or provide detection of deep vein thrombosis (DVT).

In still other embodiments, other types of cardiovascular signal sensors may be used to acquire measurements. For example, a subject's heart rate may be determined using a pulse oximeter that is coupled to a subject's finger or ear.

FIG. 11 illustrates an exemplary graphical display 1100 that can be displayed on a computer display screen 618 of a blood volume monitor 612 (FIG. 6). The display 1100 comprises an area 1102 for displaying a trend line 1104 of estimated blood volumes. By way of example, the display 1100 is configured for the display of estimated relative blood volumes. However, one of ordinary skill in the art will understand how the display 1100 may be reconfigured for the display of estimated actual blood volumes. In some embodiments, display 1100 displays the estimated relative or actual blood volume as a number or percentage rather than a trend line. In some embodiments, display 1100 displays a default value of 1 or 100% for BVI. For example, using the data in Table 1, prior to calculating the BVI #3 at measurement 10, display 1100 may display a BVI of 1 or 100%. From measurement 10 through the final measurement, display 1100 may display either the BVI as calculated (column labeled BVI #3) or as a percentage. Additionally, display 1100 may display a BVI value rounded to the nearest whole percentage point or other appropriate value. For example, the BVI at measurement 10 from Table 1 could be displayed as 99%.

The bottom axis 1106 of the display is a time axis, and in some cases may display time units spanning a fifteen minute history. Alternately, the time axis 1106 may display time units spanning a longer or shorter history. In still other cases, a blood volume monitor device may be provided with a mechanical button, switch or other selection mechanism that allows a user to select the length of the history spanned by the time axis 1106. The selection mechanism could also take the form of a graphical button or other graphical selection mechanism that is displayed as part of the graphical display 1102. Useful selectable histories may include, for example, a 15 minute, 1 hour, two hour, and five hour history (though any other set of histories may be provided). The vertical axis 1108 may be labeled with percentages or ratios corresponding to estimated relative blood volumes. Or, if the baseline blood volume of a subject is known, and if a blood volume monitor is capable of computing and displaying estimated actual blood volumes, the vertical axis 1108 may be labeled with volume units (e.g., pints or liters).

In the trend line display area 1102, 100% (or a subject's baseline relative or actual blood volume) may be displayed approximately two-thirds up on the vertical axis 1108. This is believed to be useful in that blood loss is typically more common (and can be more significant) than blood gain.

The display 1100 may take the form of a yellow fluorescent display on a liquid crystal display (LCD) or cathode ray tube (CRT), as is commonly used in an operating room. The display 1100 may also take other forms and may use other colors.

The display 1100 may also display a number 1110 indicative of the most current estimated blood volume. The most current heart rate 1112 and electrical body impedance 1114, and other parameters, may also be displayed. However, in cases where blood volume monitoring is the primary use of the display 1100, the heart rate 1112 and electrical body impedance 1114 may be displayed in a smaller font than the estimated blood volume.

When a subject's estimated blood volume falls below (or exceeds) certain thresholds, an audible alarm may be sounded. A variety of alarm circuits for providing such an alarm are well known in the art. In addition, part or all of the display 1100 (e.g., the number 1110 indicative of the most current estimated blood volume) may be caused to blink when an alarm is triggered. By way of example, an alarm might be triggered when a subject's estimated blood volume is more than 30% below its baseline, or more than 10% above its baseline. Other alarm thresholds may also be useful.

In some cases, the display 1100 may further incorporate an electrode alarm, which in some cases may take the form of an audible alarm, a displayed icon, or a dotting of the trend line 1104. The electrode alarm may be triggered, for example, when 1) relative electrical body impedance has increased to a level that suggests a loose electrode connection, or 2) a subject's heart rate cannot be reliably determined.

The above-described embodiments of methods and apparatus for estimating the blood volume of a mammalian subject are exemplary only. The following claims are not intended to be limited to these exemplary embodiments. Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. A method for estimating a relative blood volume of a mammalian subject, the relative blood volume being a unitless value indicating a percentage or ratio of the subject's blood volume at a particular time, in relation to a baseline blood volume of the subject, the method comprising:
using an electronic apparatus,

computing a blood volume index from hemodynamic data for the subject, the blood volume index defined in terms of a baseline electrical body impedance of the subject, a baseline heart rate of the subject, an electrical body impedance of the subject at a time of interest, and a heart rate of the subject at the time of interest; and
using the blood volume index to generate an output indicative of an estimated relative blood volume of the subject using a formula or model that converts the blood volume index to the ratio or display value.

2. The method of claim 1, wherein the blood volume index is defined in terms of a relative heart rate and a relative electrical body impedance, the relative heart rate being a ratio of the heart rate of the subject at the time of interest over the baseline heart rate of the subject, and the relative electrical body impedance being a ratio of the electrical body impedance of the subject at the time of interest over the baseline electrical body impedance of the subject.

3. The method of claim 1, wherein the blood volume index is defined in terms of:
1 - ((heart rate of the subject at the time of interest - baseline heart rate of the subject) / baseline heart rate of the subject + ((change in electrical body impedance of the subject at the time of interest - baseline electrical body impedance) / baseline electrical body impedance of the subject) * x) / x+1, wherein the baseline heart rate and baseline electrical body impedance are calculated as averages of a plurality of measurements taken over a period of time.

4. The method of claim 3, wherein x = 5.

5. The method of claim 3, wherein the blood volume index is defined in terms of:

$$(1 - ((\text{heart rate of the subject at the time of interest - baseline heart rate of the subject}) / \text{baseline heart rate of the subject}) + (\text{change in electrical body impedance of the subject at the time of interest}) *x) / x+1 )) * (\text{change in body temperature} * y) * (\text{change in peripheral blood flow} * z) * (\text{change in body humidity measurement} * A)* (\text{change in perfusion index} * B);$$

where x, y, z, A and B are multipliers other than one.

6. The method of claim 1, wherein the baseline electrical body impedance and the electrical body impedance at the time of interest are ankle impedances.

7. The method of claim 1, wherein the baseline electrical body impedance and the electrical body impedance at the time of interest are arm impedances.

8. The method of claim 1, wherein the blood volume index is further defined in terms of at least one of a change in body temperature of the subject, a change in a perfusion index of the subject, a change in peripheral blood flow of the subject, and a change in skin humidity of the subject.

9. Apparatus for estimating a relative blood volume of a mammalian subject, the relative blood volume being a unitless value indicating a percentage or ratio of the subject's blood volume at a particular time, in relation to a baseline blood volume of the subject, the apparatus comprising:

   a number of cardiovascular signal sensors, communicable with the subject to obtain hemodynamic measurements from the subject;
   at least one integrated circuit, operably connected with the number of cardiovascular signal sensors and configured to i) compute a blood volume index from the hemodynamic measurements, the blood volume index defined in terms of a baseline electrical body impedance of the subject, a baseline heart rate of the subject, an electrical body impedance of the subject at a time of interest, and a heart rate of the subject at the time of interest, and ii) use the blood volume index to generate an output indicative of an estimated relative blood volume of the subject using a formula or model that converts the blood volume index to the ratio or display value.

10. The apparatus of claim 9, wherein the number of cardiovascular signal sensors comprises a plurality of electrodes disposed on an arm band.

11. The apparatus of claim 9, wherein the blood volume index is further defined in terms of at least one of a change in body temperature of the subject, a change in a perfusion index of the subject, a change in peripheral blood flow of the subject, and a change in skin humidity of the subject.

12. Use of an apparatus according to claim 9 for determining a blood volume index of a mammalian subject for diagnosing a circulatory disorder, wherein the at least one integrated circuit computes the blood volume index based on a baseline electrical body impedance at an extremity of the mammalian subject and an electrical body impedance at the extremity of the mammalian subject at a time of interest.

13. Use of an apparatus according to claim 12 for determining a blood volume index of a mammalian subject for diagnosing deep vein thrombosis, wherein the at least one integrated circuit computes the blood volume index based on a baseline electrical body impedance at an extremity of the mammalian subject and an electrical body impedance at the extremity of the mammalian subject at a time of interest.

**Patentansprüche**

1. Verfahren zum Schätzen eines relativen Blutvolumens eines Säugers, wobei das relative Blutvolumen ein einheitenloser Wert ist, der einen Prozentsatz oder ein Verhältnis des Blutvolumens des Objekts zu einem bestimmten Zeitpunkt in Bezug auf einen Basiswert des Blutvolumens des Objekts angibt, wobei das Verfahren umfasst:

   Verwendung einer elektronischen Vorrichtung,
   Berechnen eines Blutvolumenindex aus hämodynamischen Daten für das Objekt, wobei der Blutvolumenindex definiert ist anhand eines Basiswerts einer elektrischen Impedanz des Körpers des Objekts, eines Basiswerts der Herzfrequenz des Objekts, einer elektrischen Impedanz des Körpers des Objekts zu einem Zeitpunkt von Interesse und einer Herzfrequenz des Objekts zu einem Zeitpunkt von Interesse; und
   Verwenden des Blutvolumenindex zum Erzeugen einer Ausgabe, die ein geschätztes relatives Blutvolumen des Objekts unter Verwendung einer Formel oder eines Modells angibt, die/das den Blutvolumenindex in das Verhältnis oder den Anzeigewert umwandelt.

2. Verfahren nach Anspruch 1, wobei der Blutvolumenindex definiert ist anhand einer relativen Herzfrequenz und einer relativen elektrischen Impedanz des Körpers, wobei die relative Herzfrequenz ein Verhältnis der Herzfrequenz des Objekts zum Zeitpunkt von Interesse gegenüber dem Basiswert der Herzfrequenz des Objekts ist und die relative elektrische Impedanz des Körpers ein Verhältnis der elektrischen Impedanz des Körpers des Objekts zum Zeitpunkt von Interesse gegenüber dem Basiswert der elektrischen Impedanz des Körpers des Objekts ist.

3. Verfahren nach Anspruch 1, wobei der Blutvolumenindex definiert ist anhand von
   1 - ((Herzfrequenz des Objekts zum Zeitpunkt von Interesse - Basiswert der Herzfrequenz des Objekts) / Basiswert der Herzfrequenz des Objekts + ((Änderung der elektrischen Impedanz des Körpers des Objekts zum Zeitpunkt von Interesse - Basiswert der elektrischen Impedanz des Körpers) / Basiswert der elektrischen Impedanz des Körpers des Objekts) * x) / x+1, wobei der Basiswert der Herzfrequenz und der Basiswert der elektrischen Impedanz des Körpers als Durchschnittswerte einer Mehrzahl von über einen Zeitraum vorgenommenen Messungen berechnet werden.

4. Verfahren nach Anspruch 3, wobei x = 5.

5. Verfahren nach Anspruch 3, wobei der Blutvolumenindex definiert ist anhand von

```
(1 – ((Herzfrequenz des Objekts zum Zeitpunkt von
Interesse – Basiswert der Herzfrequenz des
Objekts)/Basiswert der Herzfrequenz des Objekts) +
(Änderung der elektrischen Impedanz des Körpers des
Objekts zum Zeitpunkt von Interesse)*x)/x+1))*(Änderung
der Körpertemperatur * y) * (Änderung des peripheren
Blutstroms * z) * (Änderung der Messung der
Körperfeuchtigkeit * A)*(Änderung des
Perfusionsindex*B);
```

wobei x, y, z, A und B von eins unterschiedliche Multiplikatoren sind.

6. Verfahren nach Anspruch 1, wobei der Basiswert der elektrischen Impedanz des Körpers und die elektrische Impedanz des Körpers zum Zeitpunkt von Interesse Fußgelenkimpedanzen sind.

7. Verfahren nach Anspruch 1, wobei der Basiswert der elektrischen Impedanz des Körpers und die elektrische Impedanz des Körpers zum Zeitpunkt von Interesse Armimpedanzen sind.

8. Verfahren nach Anspruch 1, wobei der Blutvolumenindex ferner definiert ist anhand von mindestens einer von einer Änderung der Körpertemperatur des Objekts, einer Änderung eines Perfusionsindex des Objekts, einer Änderung des peripheren Blutstroms des Objekts und einer Änderung der Hautfeuchtigkeit des Objekts.

9. Vorrichtung zum Schätzen eines relativen Blutvolumens eines Säugers, wobei das relative Blutvolumen ein einheitenloser Wert ist, der einen Prozentsatz oder ein Verhältnis des Blutvolumens des Objekts zu einem bestimmten Zeitpunkt in Bezug auf einen Basiswert des Blutvolumens des Objekts angibt, wobei die Vorrichtung umfasst:

eine Anzahl von kardiovaskulären Signalsensoren, die mit dem Objekt kommunizieren können, um hämodynamische Messungen von dem Objekt zu erhalten;
mindestens eine integrierte Schaltung, wirkverbunden mit der Anzahl von kardiovaskulären Signalsensoren und ausgelegt zum i) Berechnen eines Blutvolumenindex aus den hämodynamischen Messungen, wobei der Blutvolumenindex definiert ist anhand eines Basiswerts einer elektrischen Impedanz des Körpers des Objekts, eines Basiswert der Herzfrequenz des Objekts, einer elektrischen Impedanz des Körpers des Objekts zu einem Zeitpunkt von Interesse und einer Herzfrequenz des Objekts zu dem Zeitpunkt von Interesse, und ii) Verwenden des Blutvolumenindex zum Erzeugen einer Ausgabe, die ein geschätztes relatives Blutvolumen des Objekts unter Verwendung einer Formel oder eines Modells angibt, die/das den Blutvolumenindex in das Verhältnis oder den Anzeigewert umwandelt.

10. Vorrichtung nach Anspruch 9, wobei die Anzahl kardiovaskulärer Signalsensoren eine Mehrzahl von an einem Armband angeordneten Elektroden umfasst.

11. Vorrichtung nach Anspruch 9, wobei der Blutvolumenindex ferner definiert ist anhand von mindestens einer von einer Änderung der Körpertemperatur des Objekts, einer Änderung eines Perfusionsindex des Objekts, einer Änderung des peripheren Blutstroms des Objekts und einer Änderung der Hautfeuchtigkeit des Objekts.

12. Verwenden einer Vorrichtung nach Anspruch 9 zum Ermitteln eines Blutvolumenindex eines Säugers zur Diagnose einer Durchblutungsstörung, wobei die mindestens eine integrierte Schaltung den Blutvolumenindex berechnet, basierend auf einem Basiswert einer elektrischen Impedanz des Körpers an einer Extremität des Säugers und einer elektrischen Impedanz des Körpers an der Extremität des Säugers zu einem Zeitpunkt von Interesse.

13. Verwenden einer Vorrichtung nach Anspruch 12 zum Ermitteln eines Blutvolumenindex eines Säugers zur Diagnose einer tiefen Venenthrombose, wobei die mindestens eine integrierte Schaltung den Blutvolumenindex berechnet, basierend auf einem Basiswert einer elektrischen Impedanz des Körpers an einer Extremität des Säugers und einer elektrischen Impedanz des Körpers an der Extremität des Säugers zu einem Zeitpunkt von Interesse.

**Revendications**

1. Procédé permettant d'estimer un volume sanguin relatif d'un sujet mammifère, le volume sanguin relatif étant une valeur sans unité indiquant un pourcentage ou un rapport du volume sanguin du sujet à un moment particulier, en relation avec un volume sanguin de référence du sujet, le procédé consistant à :

utiliser un appareil électronique,
calculer un indice volumique sanguin à partir de données hémodynamiques pour le sujet, l'indice volumique sanguin étant défini en termes d'une impédance corporelle électrique de référence du sujet, d'une fréquence cardiaque de référence du sujet, d'une impédance corporelle électrique du sujet à un moment d'intérêt et d'une fréquence cardiaque du sujet au moment d'intérêt ; et
utiliser l'indice volumique sanguin pour générer une sortie indiquant un volume sanguin relatif estimé du sujet à l'aide d'une formule ou d'un modèle qui convertit l'indice volumique sanguin en rapport ou valeur d'affichage.

2. Procédé selon la revendication 1, dans lequel l'indice volumique sanguin est défini en termes d'une fréquence cardiaque relative et d'une impédance corporelle électrique relative, la fréquence cardiaque relative étant un rapport de la fréquence cardiaque du sujet au moment d'intérêt sur la fréquence cardiaque de référence du sujet, et l'impédance corporelle électrique relative étant un rapport de l'impédance corporelle électrique du sujet au moment d'intérêt sur l'impédance corporelle électrique de référence du sujet.

3. Procédé selon la revendication 1, dans lequel l'indice volumique sanguin est défini en termes de :
1 - ((fréquence cardiaque du sujet au moment d'intérêt - fréquence cardiaque de référence du sujet) / fréquence cardiaque de référence du sujet + ((variation de l'impédance corporelle électrique du sujet au moment d'intérêt - impédance corporelle électrique de référence) / impédance corporelle électrique de référence du sujet) * x) / x+1, où la fréquence cardiaque de référence et l'impédance corporelle électrique de référence sont calculées en tant que moyennes d'une pluralité de mesures prises sur une période de temps.

**4.** Procédé selon la revendication 3, dans lequel x = 5.

**5.** Procédé selon la revendication 3, dans lequel l'indice volumique sanguin est défini en termes de :

```
(1 - ((fréquence cardiaque du sujet au moment d'intérêt
- fréquence cardiaque de référence du sujet) /
fréquence cardiaque de référence du sujet) + (variation
de l'impédance corporelle électrique du sujet au moment
d'intérêt) *x) / x+1)) * (variation de la température
corporelle  *  y)  * (variation  du  flux  sanguin
périphérique  *  z)  * (variation  de  la  mesure  de
l'humidité corporelle * A)* (variation de l'indice de
perfusion * B) ;
```

où x, y, z, A et B sont des multiplicateurs différents de un.

**6.** Procédé selon la revendication 1, dans lequel l'impédance corporelle électrique de référence et l'impédance corporelle électrique au moment d'intérêt sont des impédances de cheville.

**7.** Procédé selon la revendication 1, dans lequel l'impédance corporelle électrique de référence et l'impédance corporelle électrique au moment d'intérêt sont des impédances de bras.

**8.** Procédé selon la revendication 1, dans lequel l'indice volumique sanguin est en outre défini en termes d'une variation de la température corporelle du sujet et/ou d'une variation d'un indice de perfusion du sujet et/ou d'une variation du flux sanguin périphérique du sujet et/ou d'une variation de l'humidité cutanée du sujet.

**9.** Appareil permettant d'estimer un volume sanguin relatif d'un sujet mammifère, le volume sanguin relatif étant une valeur sans unité indiquant un pourcentage ou un rapport du volume sanguin du sujet à un moment particulier, en relation avec un volume sanguin de référence du sujet, l'appareil comprenant :

un certain nombre de capteurs de signaux cardiovasculaires, pouvant communiquer avec le sujet pour obtenir des mesures hémodynamiques du sujet ; au moins un circuit intégré, connecté fonctionnellement avec le certain nombre de capteurs de signaux cardiovasculaires et configuré pour i) calculer un indice volumique sanguin à partir des mesures hémodynamiques, l'indice volumique sanguin étant défini en termes d'une impédance corporelle électrique de référence du sujet, d'une fréquence cardiaque de référence du sujet, d'une impédance corporelle électrique du sujet à un moment d'intérêt et d'une fréquence cardiaque du sujet au moment d'intérêt, et pour ii) utiliser l'indice volumique sanguin pour générer une sortie indiquant un volume sanguin relatif estimé du sujet à l'aide d'une formule ou d'un modèle qui convertit l'indice volumique sanguin en rapport ou valeur d'affichage.

**10.** Appareil selon la revendication 9, dans lequel le certain nombre de capteurs de signaux cardiovasculaires comprend une pluralité d'électrodes disposées sur un brassard.

**11.** Appareil selon la revendication 9, dans lequel l'indice volumique sanguin est en outre défini en termes d'une variation de la température corporelle du sujet et/ou d'une variation d'un indice de perfusion du sujet et/ou d'une variation du flux sanguin périphérique du sujet et/ou d'une variation de l'humidité cutanée du sujet.

**12.** Utilisation d'un appareil selon la revendication 9 pour déterminer un indice volumique sanguin d'un sujet mammifère pour le diagnostic d'un trouble circulatoire, dans laquelle l'au moins un circuit intégré calcule l'indice volumique sanguin sur la base d'une impédance corporelle électrique de référence au niveau d'une extrémité du sujet mammifère et d'une impédance corporelle électrique au niveau de l'extrémité du sujet mammifère à un moment d'intérêt.

13. Utilisation d'un appareil selon la revendication 12 pour déterminer un indice volumique sanguin d'un sujet mammifère pour le diagnostic d'une thrombose veineuse profonde, dans laquelle l'au moins un circuit intégré calcule l'indice volumique sanguin sur la base d'une impédance corporelle électrique de référence au niveau d'une extrémité du sujet mammifère et d'une impédance corporelle électrique au niveau de l'extrémité du sujet mammifère à un moment d'intérêt.

| CBV (ml) | T15 (Ω) | T100 (Ω) | HR (bpm) | MAP (mmHg) | CBV% | T15% | T100% | HR% | MAP% | BVI #1 | BVI #2 |
|---|---|---|---|---|---|---|---|---|---|---|---|
| 1199 | 53.8 | 43.4 | 75 | 104 | 71% | 107% | 108% | 110% | 95% | 1.190737 | 1.541923 |
| 1288 | 52.5 | 42.0 | 73 | 108 | 76% | 105% | 104% | 107% | 98% | 1.121598 | 1.313872 |
| 1447 | 50.9 | 41.2 | 72 | 113 | 86% | 102% | 102% | 106% | 103% | 1.085162 | 1.190518 |
| 1554 | 52.7 | 42.1 | 58 | 110 | 92% | 105% | 105% | 85% | 100% | 0.893254 | 1.054507 |
| 1591 | 50.1 | 40.6 | 65 | 110 | 94% | 100% | 101% | 96% | 100% | 0.965394 | 1.003439 |
| 1613 | 52.2 | 41.7 | 55 | 107 | 96% | 104% | 104% | 81% | 97% | 0.839004 | 0.959723 |
| 1639 | 50.0 | 40.3 | 78 | 106 | 97% | 100% | 100% | 115% | 96% | 1.149912 | 1.161326 |
| 1689 | 50.1 | 40.2 | 68 | 110 | 100% | 100% | 100% | 100% | 100% | 1.000000 | 1.000000 |

FIG. 1A

| CBV (ml) | T15 (Ω) | T100 (Ω) | HR (bpm) | MAP (mmHg) | CBV% | T15% | T100% | HR% | MAP% | BVI #1 | BVI #2 |
|---|---|---|---|---|---|---|---|---|---|---|---|
| 1509 | 42.8 | 30.0 | 65 | 92 | 75% | 106% | 106% | 148% | 97% | 1.566013 | 1.920977 |
| 1556 | 42.2 | 29.8 | 58 | 92 | 78% | 104% | 105% | 132% | 97% | 1.388050 | 1.667523 |
| 1636 | 41.3 | 29.2 | 55 | 95 | 82% | 102% | 103% | 125% | 100% | 1.289753 | 1.448763 |
| 1803 | 41.0 | 28.8 | 62 | 101 | 90% | 101% | 102% | 141% | 106% | 1.433987 | 1.533569 |
| 1824 | 40.7 | 28.7 | 55 | 95 | 91% | 100% | 101% | 125% | 100% | 1.267668 | 1.338339 |
| 1908 | 41.5 | 29.0 | 57 | 97 | 95% | 102% | 102% | 130% | 102% | 1.327498 | 1.455670 |
| 1999 | 40.5 | 28.3 | 44 | 95 | 100% | 100% | 100% | 100% | 100% | 1.000000 | 1.000000 |
| 2034 | 40.3 | 28.3 | 62 | 97 | 102% | 100% | 100% | 141% | 102% | 1.409091 | 1.409091 |

FIG. 1B

| CBV (ml) | T15 (Ω) | T100 (Ω) | HR (bpm) | MAP (mmHg) | CBV% | T15% | T100% | HR% | MAP% | BVI #1 | BVI #2 |
|---|---|---|---|---|---|---|---|---|---|---|---|
| 1250 | 54.8 | 42.0 | 80 | 85 | 74% | 106% | 113% | 105% | 100% | 1.191658 | 1.747766 |
| 1299 | 53.7 | 41.1 | 79 | 83 | 77% | 104% | 111% | 104% | 98% | 1.151546 | 1.599837 |
| 1337 | 53.0 | 40.8 | 78 | 98 | 79% | 103% | 110% | 103% | 115% | 1.128671 | 1.538091 |
| 1681 | 51.8 | 38.2 | 74 | 82 | 100% | 100% | 103% | 97% | 96% | 1.002554 | 1.538091 |
| 1682 | 51.6 | 37.1 | 76 | 85 | 100% | 100% | 100% | 100% | 100% | 1.000000 | 1.118031 |
| 1693 | 52.2 | 39.2 | 67 | 88 | 101% | 101% | 106% | 88% | 104% | 0.931480 | 1.000000 |
| 1715 | 51.5 | 37.0 | 70 | 81 | 102% | 100% | 100% | 92% | 95% | 0.918570 | 1.131082 |
| 1733 | 52.8 | 40.4 | 57 | 83 | 103% | 102% | 109% | 75% | 98% | 0.816712 | 0.908640 |
| | | | | | | | | | | | 1.083558 |

## FIG. 1C

| CBV (ml) | T15 (Ω) | T100 (Ω) | HR (bpm) | MAP (mmHg) | CBV% | T15% | T100% | HR% | MAP% | BVI #1 | BVI #2 |
|---|---|---|---|---|---|---|---|---|---|---|---|
| 1659 | 53.5 | 39.7 | 64 | 80 | 73% | 106% | 106% | 133% | 99% | 1.419124 | 1.762288 |
| 1814 | 52.2 | 38.9 | 51 | 83 | 79% | 103% | 104% | 106% | 102% | 1.108076 | 1.290382 |
| 1834 | 50.9 | 37.8 | 46 | 80 | 80% | 100% | 101% | 96% | 99% | 0.971180 | 1.022565 |
| 2177 | 53.0 | 39.4 | 48 | 84 | 95% | 105% | 106% | 100% | 104% | 1.056300 | 1.281501 |
| 2254 | 50.6 | 37.5 | 47 | 88 | 99% | 100% | 101% | 98% | 109% | 0.984417 | 1.005418 |
| 2275 | 53.0 | 39.5 | 45 | 88 | 100% | 105% | 106% | 94% | 109% | 0.992795 | 1.213975 |
| 2282 | 50.7 | 37.3 | 48 | 81 | 100% | 100% | 100% | 100% | 100% | 1.000000 | 1.000000 |
| 2317 | 49.8 | 36.6 | 50 | 78 | 102% | 98% | 98% | 104% | 96% | 1.022118 | 0.943923 |

## FIG. 1D

EP 2 592 999 B1

| CBV (ml) | T15 (Ω) | T100 (Ω) | HR (bpm) | MAP (mmHg) | CBV% | T15% | T100% | HR% | MAP% | BVI #1 | BVI #2 |
|---|---|---|---|---|---|---|---|---|---|---|---|
| 1269 | 48.7 | 36.9 | 75 | 100 | 81% | 109% | 109% | 123% | 102% | 1.342274 | 1.793336 |
| 1332 | 47.5 | 35.6 | 68 | 98 | 85% | 107% | 105% | 111% | 100% | 1.274120 | 1.411582 |
| 1398 | 47.4 | 35.6 | 67 | 96 | 89% | 107% | 105% | 110% | 98% | 1.156853 | 1.390824 |
| 1418 | 45.8 | 33.7 | 70 | 86 | 90% | 103% | 100% | 115% | 88% | 1.144146 | 1.130566 |
| 1490 | 46.4 | 34.2 | 77 | 85 | 95% | 104% | 101% | 126% | 87% | 1.277233 | 1.336987 |
| 1575 | 44.5 | 33.8 | 61 | 98 | 100% | 100% | 100% | 100% | 100% | 1.000000 | 1.000000 |
| 1642 | 46.1 | 35.0 | 62 | 104 | 104% | 104% | 104% | 102% | 106% | 1.052478 | 1.196818 |
| 1663 | 45.0 | 33.8 | 62 | 100 | 106% | 101% | 100% | 102% | 102% | 1.016393 | 1.016393 |

## FIG. 1E

| CBV (ml) | T15 (Ω) | T100 (Ω) | HR (bpm) | MAP (mmHg) | CBV% | T15% | T100% | HR% | MAP% | BVI #1 | BVI #2 |
|---|---|---|---|---|---|---|---|---|---|---|---|
| 1063 | 62.6 | 47.8 | 87 | 84 | 61% | 105% | 106% | 130% | 106% | 1.379303 | 1.702488 |
| 1246 | 62.7 | 47.6 | 98 | 85 | 71% | 105% | 106% | 146% | 108% | 1.547197 | 1.885240 |
| 1318 | 60.3 | 45.2 | 98 | 87 | 75% | 101% | 100% | 146% | 110% | 1.469187 | 1.495191 |
| 1520 | 59.9 | 45.4 | 70 | 86 | 87% | 100% | 101% | 104% | 109% | 1.054063 | 1.091211 |
| 1521 | 58.4 | 44.6 | 65 | 82 | 87% | 98% | 99% | 97% | 104% | 0.961526 | 0.927032 |
| 1560 | 59.8 | 45.0 | 67 | 81 | 89% | 100% | 100% | 100% | 103% | 1.000000 | 1.000000 |
| 1569 | 59.3 | 44.8 | 59 | 87 | 90% | 99% | 100% | 88% | 110% | 0.876683 | 0.861028 |
| 1749 | 59.8 | 45.0 | 67 | 79 | 100% | 100% | 100% | 100% | 100% | 1.000000 | 1.000000 |

## FIG. 1F

FIG. 1G

| CBV (ml) | T15 (Ω) | T100 (Ω) | HR (bpm) | MAP (mmHg) | CBV% | T15% | T100% | HR% | MAP% | BVI #1 | BVI #2 |
|---|---|---|---|---|---|---|---|---|---|---|---|
| 1143 | 57.2 | 43.6 | 65 | 77 | 71% | 107% | 109% | 114% | 77% | 1.242982 | 1.653509 |
| 1216 | 54.8 | 41.2 | 89 | 100 | 76% | 102% | 103% | 156% | 100% | 1.608246 | 1.795614 |
| 1392 | 54.2 | 40.8 | 59 | 106 | 87% | 101% | 102% | 104% | 106% | 1.055789 | 1.138596 |
| 1509 | 53.8 | 40.3 | 57 | 105 | 94% | 100% | 101% | 100% | 105% | 1.007500 | 1.037500 |
| 1565 | 53.3 | 39.5 | 55 | 104 | 97% | 99% | 99% | 96% | 104% | 0.952851 | 0.904605 |
| 1569 | 53.5 | 39.6 | 58 | 100 | 98% | 100% | 99% | 102% | 100% | 1.007368 | 0.966667 |
| 1609 | 53.6 | 40.0 | 57 | 100 | 100% | 100% | 100% | 100% | 100% | 1.000000 | 1.000000 |

FIG. 1H

| CBV (ml) | T15 (Ω) | T100 (Ω) | HR (bpm) | MAP (mmHg) | CBV% | T15% | T100% | HR% | MAP% | BVI #1 | BVI #2 |
|---|---|---|---|---|---|---|---|---|---|---|---|
| 1370 | 44.3 | 34.2 | 61 | 92 | 76% | 106% | 105% | 130% | 103% | 1.365761 | 1.637316 |
| 1517 | 42.7 | 33.2 | 49 | 92 | 84% | 102% | 102% | 104% | 103% | 1.065008 | 1.154828 |
| 1656 | 42.2 | 32.6 | 54 | 97 | 92% | 101% | 100% | 115% | 109% | 1.152471 | 1.166612 |
| 1671 | 42.1 | 32.6 | 49 | 87 | 92% | 100% | 100% | 104% | 98% | 1.045761 | 1.058592 |
| 1686 | 43.1 | 33.2 | 47 | 89 | 93% | 103% | 102% | 100% | 100% | 1.021538 | 1.107692 |
| 1809 | 41.9 | 32.5 | 47 | 89 | 100% | 100% | 100% | 100% | 100% | 1.000000 | 1.000000 |
| 1947 | 42.1 | 32.6 | 50 | 94 | 108% | 100% | 100% | 106% | 106% | 1.067103 | 1.080196 |
| 1970 | 41.6 | 32.4 | 52 | 90 | 109% | 99% | 100% | 111% | 101% | 1.102979 | 1.089362 |

22

| CBV (ml) | T15 (Ω) | T100 (Ω) | HR (bpm) | MAP (mmHg) | CBV% | T15% | T100% | HR% | MAP% | BVI #1 | BVI #2 |
|---|---|---|---|---|---|---|---|---|---|---|---|
| 1052 | 60.0 | 48.5 | 57 | 101 | 76% | 106% | 105% | 110% | 105% | 1.155727 | 1.394022 |
| 1073 | 60.3 | 48.9 | 98 | 98 | 77% | 106% | 106% | 188% | 102% | 2.003428 | 2.478679 |
| 1104 | 58.0 | 47.5 | 72 | 97 | 79% | 102% | 103% | 138% | 101% | 1.429766 | 1.610368 |
| 1149 | 59.0 | 48.4 | 83 | 108 | 83% | 104% | 105% | 160% | 113% | 1.679431 | 2.012542 |
| 1290 | 59.6 | 48.8 | 68 | 102 | 93% | 105% | 106% | 131% | 106% | 1.387291 | 1.705686 |
| 1375 | 59.1 | 47.8 | 55 | 108 | 99% | 104% | 104% | 106% | 113% | 1.099080 | 1.264632 |
| 1377 | 56.6 | 46.1 | 58 | 97 | 99% | 100% | 100% | 112% | 101% | 1.117809 | 1.127508 |
| 1389 | 56.7 | 46.0 | 52 | 96 | 100% | 100% | 100% | 100% | 100% | 1.000000 | 1.000000 |

FIG. 1I

## Subject #1

FIG. 2A

## Subject #2

FIG. 2B

## Subject #3

**FIG. 2C**

## Subject #4

**FIG. 2D**

**Subject #5**

FIG. 2E

**Subject #6**

FIG. 2F

## Subject #7

FIG. 2G

## Subject #8

FIG. 2H

Subject #9

FIG. 2I

Subject #1

FIG. 3A

## Subject #2

FIG. 3B

## Subject #3

FIG. 3C

## Subject #4

FIG. 3D

## Subject #5

FIG. 3E

**Subject #6**

FIG. 3F

**Subject #7**

FIG. 3G

## Subject #8

FIG. 3H

## Subject #9

FIG. 3I

Correlation Between BVI #2 and Estimated Relative Blood Volume for Subjects #1-9

FIG. 4

*500*

| Acquire hemodynamic measurements from a subject | *506* |

| Process the hemodynamic measurements to generate hemodynamic data | *508* |

Using an electronic apparatus:

| Compute a blood volume index from the hemodynamic data for the subject, the blood volume index defined in terms of a baseline electrical body impedance of the subject, a baseline heart rate of the subject, an electrical body impedance of the subject at a time of interest, and a heart rate of the subject at the time of interest | *502* |

| Use the blood volume index to generate an output indicative of an estimated blood volume of the subject | *504* |

FIG. 5

FIG. 6

FIG. 7

**FIG. 8**

HEMODYNAMIC DATA *810* →

802

PROCESSING UNIT *808*

COMPUTER DATA STORAGE *806*

804

ESTIMATED BLOOD VOLUME *812* →

*800*

**FIG. 9**

COMPUTER READABLE MEDIUM *900*

*902*

Instructions that, when executed by at least one processing unit, cause the at least one processing unit to: 1) compute a blood volume index from hemodynamic data for the subject, and 2) use the blood volume index to generate an output indicative of an estimated central blood volume of the subject

FIG. 10

FIG. 11

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61495843
- US 61493847 A
- US 61365270